# EUROPEAN PATENT SPECIFICATION

(11) **EP 1 409 012 B1**
(45) Date of publication and mention of the grant of the patent: **11.02.2009**
(21) Application number: 02780874.0
(22) Date of filing: 13.05.2002
(51) Int. Cl.: A61K 39/295, C12N 15/861, A61K 39/235

(54) **METHODS OF INDUCING A CYTOTOXIC IMMUNE RESPONSE AND RECOMBINANT SIMIAN ADENOVIRUS COMPOSITIONS USEFUL THEREIN**
VERFAHREN ZUR AUSLÖSUNG EINER ZYTOTOXISCHEN IMMUNANTWORT UND DAFÜR NÜTZLICHE REKOMBINANTE AFFEN-ADENOVIRUS-ZUSAMMENSETZUNGEN
PROCEDES VISANT A INDUIRE UNE REACTION IMMUNITAIRE CYTOTOXIQUE ET COMPOSITIONS D'ADENOVIRUS SIMIEN RECOMBINE UTILES A CET EFFET

(30) Priority: 22.06.2001 US 300131 P; 12.07.2001 US 304843 P
(43) Date of publication of application: 21.04.2004
(73) Proprietor: THE WISTAR INSTITUTE OF ANATOMY AND BIOLOGY, Philadelpia, PA 19104-4268 (US); The Trustees of The University of Pennsylvania, Philadelphia, Pennsylvania 19104-6283 (US)
(72) Inventor: ERTL, Hildegund, C., J., Villanova, PA 19085 (US); WILSON, James, M., Gladwyne, PA 19035 (US)
(74) Representative: Manaton, Ross Timothy
(86) International application number: PCT/US2002/015239
(87) International publication number: WO 2003/000283

(56) References cited:
- WO-A-98/10087
- US-A- 6 083 716
- XIANG ZHIQUAN ET AL: "Novel, chimpanzee serotype 68-based adenoviral vaccine carrier for induction of antibodies to a transgene product." JOURNAL OF VIROLOGY. MAR 2002, vol. 76, no. 6, March 2002 (2002-03), pages 2667-2675, XP002331618 ISSN: 0022-538X
- COHEN CHRISTOPHER J ET AL: "Chimpanzee adenovirus CV-68 adapted as a gene delivery vector interacts with the coxsackievirus and adenovirus receptor" JOURNAL OF GENERAL VIROLOGY, vol. 83, no. 1, January 2002 (2002-01), pages 151-155, XP002331619 ISSN: 0022-1317
- QIU ET AL.: 'Evaluation of novel human immunodeficiency virus type 1 Gag DNA vaccines for protein expression in mammalian cells and induction of immune responses' JOURNAL OF VIROLOGY vol. 73, no. 11, 1999, pages 9145 - 9152, XP002172801
- TOES ET AL.: 'Protective anti-tumor immunity induced by vaccination with recombinant adenovirus encoding multiple tumor-associated cytotoxic T lymphocyte epitopes in a string-of-beads fashion' PROC. NATL. ACAD. SCI. USA vol. 94, 1997, pages 14660 - 14665, XP002164428
- FARINA ET AL.: 'Replication-defective vector based on a Chimpanzee adenovirus' JOURNAL OF VIROLOGY vol. 75, no. 23, 2001, pages 11603 - 11613, XP002957497

### SEQUENCE LISTING

<110> The Wistar Institute of Anatomy and Biology
   The Trustees of The University of Pennsylvania
   Ertl, Hildegund C.J.
   Wilson, James M.
<120> Methods of Inducing a Cytotoxic Immune Response and Recombinant Simian Ad enovirus Compositions Useful Therein
<130> WST104A/UPNN2628A
<150> US 60/300,131
   <151> 2001-06-22
<150> US 60/304,843
   <151> 2001-07-12
<160> 13
<170> PatentIn version 3.1
<210> 1
   <211> 9
   <212> PRT
   <213> Artificial sequence
<220>
   <223> Synthetic peptide which carries the immunodominant CD8+ T cell ep itope for the H-2d haplotype
<400> 1
   Ala Met Gln Met Leu Lys Glu Thr Ile
   1               5
<210> 2
   <211> 19
   <212> DNA
   <213> Artificial sequence
<220>
   <223> 5' primer for the rabies virus glycoprotein
<400> 2
   aagcatttcc gcccaacac 19
<210> 3
   <211> 22
   <212> DNA
   <213> Artificial sequence
<220>
   <223> 3' primer for rabies virus glycoprotein
<400> 3
   ggttagtgga gcagtaggta ga 22
<210> 4
   <211> 25
   <212> DNA
   <213> Artificial sequence
<220>
   <223> 5' primer for glutaraldehyde-3-phosphate dehydrogenase (GAPDH)
<400> 4
   ggtgaaggtc ggtgtgaacg gattt 25
<210> 5
   <211> 25
   <212> DNA
   <213> Artificial Sequence
<220>
   <223> 3' primer for GAPDH
<400> 5
   aatgccaaag ttgtcatgga tgacc 25
<210> 6
   <211> 7228
   <212> DNA
   <213> Artificial Sequence
<220>
   <223> Modified HIV-1 gag sequence
<220>
   <221> CDS
   <222> (729) .. (1820)
   <223>
<400> 6
   tggaagggct aatttggtcc caaaaaagac aagagatcct tgatctgtgg atctaccaca    60
   cacaaggcta cttccctgat tggcagaact acacaccagg gccagggatc agatatccac   120
   tgacctttgg atggtgcttc aagttagtac cagttgaacc agagcaagta gaagaggcca   180
   aataaggaga gaagaacagc ttgttacacc ctatgagcca gcatgggatg gaggacccgg   240
   agggagaagt attagtgtgg aagtttgaca gcctcctagc atttcgtcac atggcccgag   300
   agctgcatcc ggagtactac aaagactgct gacatcgagc tttctacaag ggactttccg   360
   ctggggactt tccagggagg tgtggcctgg gcgggactgg ggagtggcga gccctcagat   420
   gctacatata agcagctgct ttttgcctgt actgggtctc tctggttaga ccagatctga    480
   gcctgggagc tctctggcta actagggaac ccactgctta agcctcaata aagcttgcct    540
   tgagtgctca aagtagtgtg tgcccgtctg ttgtgtgact ctggtaacta gagatccctc    600
   agaccctttt agtcagtgtg gaaaatctct agcagtggcg cccgaacagg gacttgaaag    660
   cgaaagtaaa gccagaggag atctctcgac gcaggactcg gcttgctgaa gcgcgcgtcg    720
   acagagag atg ggt gcg aga gcg tca gta tta agc ggg gga gaa tta gat      770
            Met Gly Ala Arg Ala Ser Val Leu Ser Gly Gly Glu Leu Asp
            1               5                   10
   cga tgg gaa aaa att cgg tta agg cca ggg gga aag aag aag tac aag      818
   Arg Trp Glu Lys Ile Arg Leu Arg Pro Gly Gly Lys Lys Lys Tyr Lys
   15                  20                  25                  30
   cta aag cac atc gta tgg gca agc agg gag cta gaa cga ttc gca gtt      866
   Leu Lys His Ile Val Trp Ala Ser Arg Glu Leu Glu Arg Phe Ala Val
                   35                  40                  45
   aat cct ggc ctg tta gaa aca tca gaa ggc tgt aga caa ata ctg gga      914
   Asn Pro Gly Leu Leu Glu Thr Ser Glu Gly Cys Arg Gln Ile Leu Gly
               50                  55                  60
   cag cta caa cca tcc ctt cag aca gga tca gag gag ctt cga tca cta      962
   Gln Leu Gln Pro Ser Leu Gln Thr Gly Ser Glu Glu Leu Arg Ser Leu
           65                  70                  75
   tac aac aca gta gca acc ctc tat tgt gtg cac cag cgg atc gag atc      1010
   Tyr Asn Thr Val Ala Thr Leu Tyr Cys Val His Gln Arg Ile Glu Ile
       80                  85                  90
   aag gac acc aag gaa gct tta gac aag ata gag gaa gag caa aac aag      1058
   Lys Asp Thr Lys Glu Ala Leu Asp Lys Ile Glu Glu Glu Gln Asn Lys
   95                  100                 105                 110
   tcc aag aag aag gcc cag cag gca gca gct gac aca gga cac agc aat      1106
   Ser Lys Lys Lys Ala Gln Gln Ala Ala Ala Asp Thr Gly His Ser Asn
                   115                 120                 125
   cag gtc agc caa aat tac cct ata gtg cag aac atc cag ggg caa atg      1154
   Gln Val Ser Gln Asn Tyr Pro Ile Val Gln Asn Ile Gln Gly Gln Met
               130                 135                 140
   gta cat cag gcc ata tca cct aga act tta aat gca tgg gta aaa gta      1202
   Val His Gln Ala Ile Ser Pro Arg Thr Leu Asn Ala Trp Val Lys Val
           145                 150                 155
   gta gaa gag aag gct ttc agc cca gaa gtg ata ccc atg ttt tca gca      1250
   Val Glu Glu Lys Ala Phe Ser Pro Glu Val Ile Pro Met Phe Ser Ala
       160                 165                 170
   tta tca gaa gga gcc acc cca cag gac ctg aac acg atg ttg aac acc      1298
   Leu Ser Glu Gly Ala Thr Pro Gln Asp Leu Asn Thr Met Leu Asn Thr
   175                 180                 185                 190
   gtg ggg gga cat caa gca gcc atg caa atg tta aaa gag acc atc aat      1346
   Val Gly Gly His Gln Ala Ala Met Gln Met Leu Lys Glu Thr Ile Asn
                   195                 200                 205
   gag gaa gct gca gaa tgg gat aga gtg cat cca gtg cat gca ggg cct      1394
   Glu Glu Ala Ala Glu Trp Asp Arg Val His Pro Val His Ala Gly Pro
               210                 215                 220
   att gca cca ggc cag atg aga gaa cca agg gga agt gac ata gca gga      1442
   Ile Ala Pro Gly Gln Met Arg Glu Pro Arg Gly Ser Asp Ile Ala Gly
           225                 230                 235
   act act agt acc ctt cag gaa caa ata gga tgg atg aca aat aat cca      1490
   Thr Thr Ser Thr Leu Gln Glu Gln Ile Gly Trp Met Thr Asn Asn Pro
       240                 245                 250
   cct atc cca gta gga gag atc tac aag agg tgg ata atc ctg gga ttg      1538
   Pro Ile Pro Val Gly Glu Ile Tyr Lys Arg Trp Ile Ile Leu Gly Leu
   255                 260                 265                 270
   aac aag atc gtg agg atg tat agc cct acc agc att ctg gac ata aga      1586
   Asn Lys Ile Val Arg Met Tyr Ser Pro Thr Ser Ile Leu Asp Ile Arg
                   275                 280                 285
   caa gga cca aag gaa ccc ttt aga gac tat gta gac cgg ttc tat aaa      1634
   Gln Gly Pro Lys Glu Pro Phe Arg Asp Tyr Val Asp Arg Phe Tyr Lys
               290                 295                 300
   act cta aga gct gag caa gct tca cag gag gta aaa aat tgg atg aca      1682
   Thr Leu Arg Ala Glu Gln Ala Ser Gln Glu Val Lys Asn Trp Met Thr
           305                 310                 315
   gaa acc ttg ttg gtc caa aat gcg aac cca gat tgt aag acc atc ctg      1730
   Glu Thr Leu Leu Val Gln Asn Ala Asn Pro Asp Cys Lys Thr Ile Leu
       320                 325                 330
   aag gct ctc ggc cca gcg gct aca cta gaa gaa atg atg aca gca tgt      1778
   Lys Ala Leu Gly Pro Ala Ala Thr Leu Glu Glu Met Met Thr Ala Cys
   335                 340                 345                 350
   cag gga gta gga gga ccc ggc cat aag gca aga gtt ttg tag              1820
   Gln Gly Val Gly Gly Pro Gly His Lys Ala Arg Val Leu
                   355                 360
   ggatccacta gttctagact cgaggggggg cccggtacct ttaagaccaa tgacttacaa    1880
   ggcagctgta gatcttagcc actttttaaa agaaaagggg ggactggaag ggctaattca    1940
   ctcccaaaga agacaagata tccttgatct gtggatctac cacacacaag gctacttccc    2000
   tgattggcag aactacacac cagggccagg ggtcagatat ccactgacct ttggatggtg    2060
   ctacaagcta gtaccagttg agccagataa ggtagaagag gccaataaag gagagaacac    2120
   cagcttgtta caccctgtga gcctgcatgg aatggatgac cctgagagag aagtgttaga    2180
   gtggaggttt gacagccgcc tagcatttca tcacgtggcc cgagagctgc atccggagta 2240
   cttcaagaac tgctgacatc gagcttgcta caagggactt tccgctgggg actttccagg 2300
   gaggcgtggc ctgggcggga ctggggagtg gcgagccctc agatgctgca tataagcagc 2360
   tgctttttgc ctgtactggg tctctctggt tagaccagat ctgagcctgg gagctctctg 2420
   gctaactagg gaacccactg cttaagcctc aataaagctt gccttgagtg cttcaagtag 2480
   tgtgtgcccg tctgttgtgt gactctggta actagagatc cctcagaccc ttttagtcag 2540
   tgtggaaaat ctctagcacc ccccaggagg tagaggttgc agtgagccaa gatcgcgcca 2600
   ctgcattcca gcctgggcaa gaaaacaaga ctgtctaaaa taataataat aagttaaggg 2660
   tattaaatat atttatacat ggaggtcata aaaatatata tatttgggct gggcgcagtg 2720
   gctcacacct gcgcccggcc ctttgggagg ccgaggcagg tggatcacct gagtttggga 2780
   gttccagacc agcctgacca acatggagaa accccttctc tgtgtatttt tagtagattt 2840
   tattttatgt gtattttatt cacaggtatt tctggaaaac tgaaactgtt tttcctctac 2900
   tctgatacca caagaatcat cagcacagag gaagacttct gtgatcaaat gtggtgggag 2960
   agggaggttt tcaccagcac atgagcagtc agttctgccg cagactcggc gggtgtcctt 3020
   cggttcagtt ccaacaccgc ctgcctggag agaggtcaga ccacagggtg agggctcagt 3080
   ccccaagaca taaacaccca agacataaac acccaacagg tccaccccgc ctgctgccca 3140
   ggcagagccg attcaccaag acgggaatta ggatagagaa agagtaagtc acacagagcc 3200
   ggctgtgcgg gagaacggag ttctattatg actcaaatca gtctccccaa gcattcgggg 3260
   atcagagttt ttaaggataa cttagtgtgt agggggccag tgagttggag atgaaagcgt 3320
   agggagtcga aggtgtcctt ttgcgccgag tcagttcctg ggtgggggcc acaagatcgg 3380
   atgagccagt ttatcaatcc gggggtgcca gctgatccat ggagtgcagg gtctgcaaaa 3440
   tatctcaagc actgattgat cttaggtttt acaatagtga tgttacccca ggaacaattt 3500
   ggggaaggtc agaatcttgt agcctgtagc tgcatgactc ctaaaccata atttcttttt 3560
   tgtttttttt tttttatttt tgagacaggg tctcactctg tcacctaggc tggagtgcag 3620
   tggtgcaatc acagctcact gcagccccta gagcggccgc caccgcggtg gagctccaat 3680
   tcgccctata gtgagtcgta ttacaattca ctggccgtcg ttttacaacg tcgtgactgg 3740
   gaaaaccctg gcgttaccca acttaatcgc cttgcagcac atcccccttt cgccagctgg 3800
   cgtaatagcg aagaggcccg caccgatcgc ccttcccaac agttgcgcag cctgaatggc 3860
   gaatggcgcg aaattgtaaa cgttaatatt ttgttaaaat tcgcgttaaa tttttgttaa 3920
   atcagctcat tttttaacca ataggccgaa atcggcaaaa tcccttataa atcaaaagaa 3980
   tagaccgaga tagggttgag tgttgttcca gtttggaaca agagtccact attaaagaac 4040
   gtggactcca acgtcaaagg gcgaaaaacc gtctatcagg gcgatggccc actacgtgaa 4100
   ccatcaccct aatcaagttt tttggggtcg aggtgccgta aagcactaaa tcggaaccct 4160
   aaagggagcc cccgatttag agcttgacgg ggaaagccgg cgaacgtggc gagaaaggaa 4220
   gggaagaaag cgaaaggagc gggcgctagg gcgctggcaa gtgtagcggt cacgctgcgc 4280
   gtaaccacca cacccgccgc gcttaatgcg ccgctacagg gcgcgtccca ggtggcactt 4340
   ttcggggaaa tgtgcgcgga acccctattt gtttattttt ctaaatacat tcaaatatgt 4400
   atccgctcat gagacaataa ccctgataaa tgcttcaata atattgaaaa aggaagagta 4460
   tgagtattca acatttccgt gtcgccctta ttcccttttt tgcggcattt tgccttcctg 4520
   tttttgctca cccagaaacg ctggtgaaag taaaagatgc tgaagatcag ttgggtgcac 4580
   gagtgggtta catcgaactg gatctcaaca gcggtaagat ccttgagagt tttcgccccg 4640
   aagaacgttt tccaatgatg agcactttta aagttctgct atgtggcgcg gtattatccc 4700
   gtattgacgc cgggcaagag caactcggtc gccgcataca ctattctcag aatgacttgg 4760
   ttgagtactc accagtcaca gaaaagcatc ttacggatgg catgacagta agagaattat 4820
   gcagtgctgc cataaccatg agtgataaca ctgcggccaa cttacttctg acaacgatcg 4880
   gaggaccgaa ggagctaacc gcttttttgc acaacatggg ggatcatgta actcgccttg 4940
   atcgttggga accggagctg aatgaagcca taccaaacga cgagcgtgac accacgatgc 5000
   ctgtagcaat ggcaacaacg ttgcgcaaac tattaactgg cgaactactt actctagctt 5060
   cccggcaaca attaatagac tggatggagg cggataaagt tgcaggacca cttctgcgct 5120
   cggcccttcc ggctggctgg tttattgctg ataaatctgg agccggtgag cgtgggtctc 5180
   gcggtatcat tgcagcactg gggccagatg gtaagccctc ccgtatcgta gttatctaca 5240
   cgacggggag tcaggcaact atggatgaac gaaatagaca gatcgctgag ataggtgcct 5300
   cactgattaa gcattggtaa ctgtcagacc aagtttactc atatatactt tagattgatt 5360
   taaaacttca tttttaattt aaaaggatct aggtgaagat cctttttgat aatctcatga 5420
   ccaaaatccc ttaacgtgag ttttcgttcc actgagcgtc agaccccgta gaaaagatca 5480
   aaggatcttc ttgagatcct ttttttctgc gcgtaatctg ctgcttgcaa acaaaaaaac 5540
   caccgctacc agcggtggtt tgtttgccgg atcaagagct accaactctt tttccgaagg 5600
   taactggctt cagcagagcg cagataccaa atactgtcct tctagtgtag ccgtagttag 5660
   gccaccactt caagaactct gtagcaccgc ctacatacct cgctctgcta atcctgttac 5720
   cagtggctgc tgccagtggc gataagtcgt gtcttaccgg gttggactca agacgatagt 5780
   taccggataa ggcgcagcgg tcgggctgaa cggggggttc gtgcacacag cccagcttgg 5840
   agcgaacgac ctacaccgaa ctgagatacc tacagcgtga gctatgagaa agcgccacgc 5900
   ttcccgaagg gagaaaggcg gacaggtatc cggtaagcgg cagggtcgga acaggagagc 5960
   gcacgaggga gcttccaggg ggaaacgcct ggtatcttta tagtcctgtc gggtttcgcc 6020
   acctctgact tgagcgtcga tttttgtgat gctcgtcagg ggggcggagc ctatggaaaa 6080
   acgccagcaa cgcggccttt ttacggttcc tggccttttg ctggcctttt gctcacatgt 6140
   tctttcctgc gttatcccct gattctgtgg ataaccgtat taccgccttt gagtgagctg 6200
   ataccgctcg ccgcagccga acgaccgagc gcagcgagtc agtgagcgag gaagcggaag 6260
   agcgcccaat acgcaaaccg cctctccccg cgcgttggcc gattcattaa tgcagctggc 6320
   acgacaggtt tcccgactgg aaagcgggca gtgagcgcaa cgcaattaat gtgagttagc 6380
   tcactcatta ggcaccccag gctttacact ttatgcttcc ggctcgtatg ttgtgtggaa 6440
   ttgtgagcgg ataacaattt cacacaggaa acagctatga ccatgattac gccaagctcg 6500
   gaattaaccc tcactaaagg gaacaaaagc tgctgcaggg tccctaactg ccaagcccca 6560
   cagtgtgccc tgaggctgcc ccttccttct agcggctgcc cccactcggc tttgctttcc 6620
   ctagtttcag ttacttgcgt tcagccaagg tctgaaacta ggtgcgcaca gagcggtaag 6680
   actgcgagag aaagagacca gctttacagg gggtttatca cagtgcaccc tgacagtcgt 6740
   cagcctcaca gggggtttat cacattgcac cctgacagtc gtcagcctca cagggggttt 6800
   atcacagtgc acccttacaa tcattccatt tgattcacaa tttttttagt ctctactgtg 6860
   cctaacttgt aagttaaatt tgatcagagg tgtgttccca gaggggaaaa cagtatatac 6920
   agggttcagt actatcgcat ttcaggcctc cacctgggtc ttggaatgtg tcccccgagg 6980
   ggtgatgact acctcagttg gatctccaca ggtcacagtg acacaagata accaagacac 7040
   ctcccaaggc taccacaatg ggccgccctc cacgtgcaca tggccggagg aactgccatg 7100
   tcggaggtgc aagcacacct gcgcatcaga gtccttggtg tggagggagg gaccagcgca 7160
   gcttccagcc atccacctga tgaacagaac ctagggaaag ccccagttct acttacacca 7220
   ggaaaggc 7228
<210> 7
   <211> 363
   <212> PRT
   <213> Artificial Sequence
<220>
   <223> Modified HIV-1 gag sequence
<400> 7
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Asn Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
   195 200 205
   Ala Ala Glu Trp Asp Arg Val His Pro Val His Ala Gly Pro Ile Ala
   210 215 220
   Pro Gly Gln Met Arg Glu Pro Arg Gly Ser Asp Ile Ala Gly Thr Thr
   225 230 235 240
   Ser Thr Leu Gln Glu Gln Ile Gly Trp Met Thr Asn Asn Pro Pro Ile
   245 250 255
   Pro Val Gly Glu Ile Tyr Lys Arg Trp Ile Ile Leu Gly Leu Asn Lys
   260 265 270
   Ile Val Arg Met Tyr Ser Pro Thr Ser Ile Leu Asp Ile Arg Gln Gly
   275 280 285
   Pro Lys Glu Pro Phe Arg Asp Tyr Val Asp Arg Phe Tyr Lys Thr Leu
   290 295 300
   Arg Ala Glu Gln Ala Ser Gln Glu Val Lys Asn Trp Met Thr Glu Thr
   305 310 315 320
   Leu Leu Val Gln Asn Ala Asn Pro Asp Cys Lys Thr Ile Leu Lys Ala
   325 330 335
   Leu Gly Pro Ala Ala Thr Leu Glu Glu Met Met Thr Ala Cys Gln Gly
   340 345 350
   Val Gly Gly Pro Gly His Lys Ala Arg Val Leu
   355 360
<210> 8
   <211> 311
   <212> PRT
   <213> Chimpanzee type adenovirus
<400> 8
   Asn Thr Cys Gln Trp Thr Tyr Lys Ala Asp Gly Glu Thr Ala Thr Glu
   1 5 10 15
   Lys Thr Tyr Thr Tyr Gly Asn Ala Pro Val Gln Gly Ile Asn Ile Thr
               20                  25                  30
   Lys Asp Gly Ile Gln Leu Gly Thr Asp Thr Asp Asp Gln Pro Ile Tyr
           35                  40                  45
   Ala Asp Lys Thr Tyr Gln Pro Glu Pro Gln Val Gly Asp Ala Glu Trp
       50                  55                  60
   His Asp Ile Thr Gly Thr Asp Glu Lys Tyr Gly Gly Arg Ala Leu Lys
   65                  70                  75                  80
   Pro Asp Thr Lys Met Lys Pro Cys Tyr Gly Ser Phe Ala Lys Pro Thr
                   85                  90                  95
   Asn Lys Glu Gly Gly Gln Ala Asn Val Lys Thr Gly Thr Gly Thr Thr
               100                 105                 110
   Lys Glu Tyr Asp Ile Asp Met Ala Phe Phe Asp Asn Arg Ser Ala Ala
           115                 120                 125
   Ala Ala Gly Leu Ala Pro Glu Ile Val Leu Tyr Thr Glu Asn Val Asp
       130                 135                 140
   Leu Glu Thr Pro Asp Thr His Ile Val Tyr Lys Ala Gly Thr Asp Asp
   145                 150                 155                 160
   Ser Ser Ser Ser Ile Asn Leu Gly Gln Gln Ala Met Pro Asn Arg Pro
                   165                 170                 175
   Val Tyr Ile Gly Phe Arg Asp Asn Phe Ile Gly Leu Met Tyr Tyr Asn
               180                 185                 190
   Ser Thr Gly Asn Met Gly Val Leu Ala Gly Gln Ala Ser Gln Leu Asn
           195                 200                 205
   Ala Val Val Asp Leu Gln Asp Arg Asn Thr Glu Leu Ser Tyr Gln Leu
       210                 215                 220
   Leu Leu Asp Ser Leu Gly Asp Arg Thr Arg Tyr Phe Ser Met Trp Asn
   225                 230                 235                 240
   Gln Ala Val Asp Ser Tyr Asp Pro Asp Val Arg Ile Ile Glu Asn His
                   245                     250                     255
   Gly Val Glu Asp Glu Leu Pro Asn Tyr Cys Phe Pro Leu Asp Ala Val
               260                     265                     270
   Gly Arg Thr Asp Thr Tyr Gln Gly Ile Lys Ala Asn Gly Thr Asp Gln
           275                     280                     285
   Thr Thr Trp Thr Lys Asp Asp Ser Val Asn Asp Ala Asn Glu Ile Gly
       290                     295                     300
   Lys Gly Asn Pro Phe Ala Met
   305                     310
<210> 9
   <211> 314
   <212> PRT
   <213> Human adenovirus type 4
<400> 9
   Asn Thr Cys Gln Trp Lys Asp Ser Asp Ser Lys Met His Thr Phe Gly
   1               5                   10                  15
   Ala Ala Ala Met Pro Gly Val Thr Gly Lys Lys Ile Glu Ala Asp Gly
               20                  25                  30
   Leu Pro Ile Arg Ile Asp Ser Thr Ser Gly Thr Asp Thr Val Ile Tyr
           35                  40                  45
   Ala Asp Lys Thr Phe Gln Pro Glu Pro Gln Val Gly Asn Asp Ser Trp
       50                  55                  60
   Val Asp Thr Asn Gly Ala Glu Glu Lys Tyr Gly Gly Arg Ala Leu Lys
   65                  70                  75                  80
   Asp Thr Thr Lys Met Asn Pro Cys Tyr Gly Ser Phe Ala Lys Pro Thr
                   85                  90                  95
   Asn Lys Glu Gly Gly Gln Ala Asn Leu Lys Asp Ser Glu Pro Ala Ala
               100                 105                 110
   Thr Thr Pro Asn Tyr Asp Ile Asp Leu Ala Phe Phe Asp Ser Lys Thr
           115                 120                 125
   Ile Val Ala Asn Tyr Asp Pro Asp Ile Val Met Tyr Thr Glu Asn Val
   130 135 140
   Asp Leu Gln Thr Pro Asp Thr His Ile Val Tyr Lys Pro Gly Thr Glu
   145 150 155 160
   Asp Thr Ser Ser Glu Ser Asn Leu Gly Gln Gln Ala Met Pro Asn Arg
   165 170 175
   Pro Asn Tyr Ile Gly Phe Arg Asp Asn Phe Ile Gly Leu Met Tyr Tyr
   180 185 190
   Asn Ser Thr Gly Asn Met Gly Val Leu Ala Gly Gln Ala Ser Gln Leu
   195 200 205
   Asn Ala Val Val Asp Leu Gln Asp Arg Asn Thr Glu Leu Ser Tyr Gln
   210 215 220
   Leu Leu Leu Asp Ser Leu Gly Asp Arg Thr Arg Tyr Phe Ser Met Trp
   225 230 235 240
   Asn Gln Ala Val Asp Ser Tyr Asp Pro Asp Val Arg Ile Ile Glu Asn
   245 250 255
   His Gly Val Glu Asp Glu Leu Pro Asn Tyr Cys Phe Pro Leu Asn Gly
   260 265 270
   Val Gly Leu Thr Asp Thr Tyr Gln Gly Val Lys Val Lys Thr Asp Ala
   275 280 285
   Gly Ser Glu Lys Trp Asp Lys Asp Asp Thr Thr Val Ser Asn Ala Asn
   290 295 300
   Glu Ile His Val Gly Asn Pro Phe Ala Met
   305 310
<210> 10
   <211> 318
   <212> PRT
   <213> Human adenovirus type 16
<400> 10
   Asn Thr Cys Gln Trp Lys Asp Ser Asp Ser Lys Met His Thr Phe Gly
   1               5                   10                  15
   Val Ala Ala Met Pro Gly Val Thr Gly Lys Lys Ile Glu Ala Asp Gly
               20                  25                  30
   Leu Pro Ile Gly Ile Asp Ser Thr Ser Gly Thr Asp Thr Val Ile Tyr
           35                  40                  45
   Ala Asp Lys Thr Phe Gln Pro Glu Pro Gln Val Gly Asn Ala Ser Trp
       50                  55                  60
   Val Asp Ala Asn Gly Thr Glu Glu Lys Tyr Gly Gly Arg Ala Leu Lys
   65                  70                  75                  80
   Asp Thr Thr Lys Met Lys Pro Cys Tyr Gly Ser Phe Ala Lys Pro Thr
                   85                  90                  95
   Asn Lys Glu Gly Gly Gln Ala Asn Leu Lys Asp Ser Glu Thr Ala Ala
               100                 105                 110
   Thr Thr Pro Asn Tyr Asp Ile Asp Leu Ala Phe Phe Asp Asn Lys Asn
           115                 120                 125
   Ile Ala Ala Asn Tyr Asp Pro Asp Ile Val Met Tyr Thr Glu Asn Val
       130                 135                 140
   Asp Leu Gln Thr Pro Asp Thr His Ile Val Tyr Lys Pro Gly Thr Glu
   145                 150                 155                 160
   Asp Thr Ser Ser Glu Ser Asn Leu Gly Gln Gln Ala Met Pro Asn Arg
                   165                 170                 175
   Pro Asn Tyr Ile Gly Phe Arg Asp Asn Phe Ile Gly Leu Met Tyr Tyr
               180                 185                 190
   Asn Ser Thr Gly Asn Met Gly Val Leu Ala Gly Gln Ala Ser Gln Leu
           195                 200                 205
   Asn Ala Val Val Asp Leu Gln Asp Arg Asn Thr Glu Leu Ser Tyr Gln
       210                 215                 220
   Leu Leu Leu Asp Ser Leu Gly Asp Arg Thr Arg Tyr Phe Ser Met Trp
   225                 230                 235                 240
   Asn Gln Ala Val Asp Ser Tyr Asp Pro Asp Val Arg Ile Ile Glu Asn
   245 250 255
   His Gly Val Glu Asp Glu Leu Pro Asn Tyr Cys Phe Pro Leu Asn Gly
   260 265 270
   Val Gly Phe Thr Asp Thr Tyr Gln Gly Val Lys Val Lys Thr Asp Ala
   275 280 285
   Val Ala Gly Thr Ser Gly Thr Gln Trp Asp Lys Asp Asp Thr Thr Val
   290 295 300
   Ser Thr Ala Asn Glu Ile His Gly Gly Asn Pro Phe Ala Met
   305 310 315
<210> 11
   <211> 323
   <212> PRT
   <213> Human adenovirus type 3
<400> 11
   Asn Thr Ser Gln Trp Ile Val Thr Thr Asn Gly Asp Asn Ala Val Thr
   1 5 10 15
   Thr Thr Thr Asn Thr Phe Gly Ile Ala Ser Met Lys Gly Gly Asn Ile
   20 25 30
   Thr Lys Glu Gly Leu Gln Ile Gly Lys Asp Ile Thr Thr Thr Glu Gly
   35 40 45
   Glu Glu Lys Pro Ile Tyr Ala Asp Lys Thr Tyr Gln Pro Glu Pro Gln
   50 55 60
   Val Gly Glu Glu Ser Trp Thr Asp Thr Asp Gly Thr Asn Glu Lys Phe
   65 70 75 80
   Gly Gly Arg Ala Leu Lys Pro Ala Thr Asn Met Lys Pro Cys Tyr Gly
   85 90 95
   Ser Phe Ala Arg Pro Thr Asn Ile Lys Gly Gly Gln Ala Lys Asn Arg
   100 105 110
   Lys Val Lys Pro Thr Thr Glu Gly Gly Val Glu Thr Glu Glu Pro Asp
   115 120 125
   Ile Asp Met Glu Phe Phe Asp Gly Arg Asp Ala Val Ala Gly Ala Leu
   130 135 140
   Ala Pro Glu Ile Val Leu Tyr Thr Glu Asn Val Asn Leu Glu Thr Pro
   145 150 155 160
   Asp Ser His Val Val Tyr Lys Pro Glu Thr Ser Asn Asn Ser His Ala
   165 170 175
   Asn Leu Gly Gln Gln Ala Met Pro Asn Arg Pro Asn Tyr Ile Gly Phe
   180 185 190
   Arg Asp Asn Phe Val Gly Leu Met Tyr Tyr Asn Ser Thr Gly Asn Met
   195 200 205
   Gly Val Leu Ala Gly Gln Ala Ser Gln Leu Asn Ala Val Val Asp Leu
   210 215 220
   Gln Asp Arg Asn Thr Glu Leu Ser Tyr Gln Leu Leu Leu Asp Ser Leu
   225 230 235 240
   Gly Asp Arg Thr Arg Tyr Phe Ser Met Trp Asn Gln Ala Val Asp Ser
   245 250 255
   Tyr Asp Pro Asp Val Arg Ile Ile Glu Asn His Gly Ile Glu Asp Glu
   260 265 270
   Leu Pro Asn Tyr Cys Phe Pro Leu Asn Gly Ile Gly Pro Gly His Thr
   275 280 285
   Tyr Gln Gly Ile Lys Lys Val Lys Thr Asp Asp Thr Asn Gly Trp Glu
   290 295 300
   Lys Asp Ala Asn Val Ala Pro Ala Asn Glu Ile Thr Ile Gly Asn Asn
   305 310 315 320
   Leu Ala Met
<210> 12
   <211> 315
   <212> PRT
   <213> Human adenovirus type 7
<400> 12
   Asn Thr Ser Gln Trp Ile Val Thr Ala Gly Glu Glu Arg Ala Val Thr
   1               5                   10                  15
   Thr Thr Thr Asn Thr Phe Gly Ile Ala Ser Met Lys Gly Asp Asn Ile
               20                  25                  30
   Thr Lys Glu Gly Leu Glu Ile Gly Lys Asp Ile Thr Ala Asp Asn Lys
           35                  40                  45
   Pro Ile Tyr Ala Asp Lys Thr Tyr Gln Pro Glu Pro Gln Val Gly Glu
       50                  55                  60
   Glu Ser Trp Thr Asp Thr Asp Gly Thr Asn Glu Lys Phe Gly Gly Arg
   65                  70                  75                  80
   Ala Leu Lys Pro Ala Thr Lys Met Lys Pro Cys Tyr Gly Ser Phe Ala
                   85                  90                  95
   Arg Pro Thr Asn Ile Lys Gly Gly Gln Ala Lys Asn Arg Lys Val Lys
               100                 105                 110
   Pro Thr Glu Gly Asp Val Glu Thr Glu Glu Pro Asp Ile Asp Met Glu
           115                 120                 125
   Phe Phe Asp Gly Arg Glu Ala Ala Asp Ala Phe Ser Pro Glu Ile Val
       130                 135                 140
   Leu Tyr Thr Glu Asn Val Asn Leu Glu Thr Pro Asp Ser His Val Val
   145                 150                 155                 160
   Tyr Lys Pro Gly Thr Ser Asp Asp Asn Ser His Ala Asn Leu Gly Gln
                   165                 170                 175
   Gln Ala Met Pro Asn Arg Pro Asn Tyr Ile Gly Phe Arg Asp Asn Phe
               180                 185                 190
   Val Gly Leu Met Tyr Tyr Asn Ser Thr Gly Asn Met Gly Val Leu Ala
           195                 200                 205
   Gly Gln Ala Ser Gln Leu Asn Ala Val Val Asp Leu Gln Asp Arg Asn
       210                 215                 220
   Thr Glu Leu Ser Tyr Gln Leu Leu Leu Asp Ser Leu Gly Asp Arg Thr
   225                 230                 235                 240
   Arg Tyr Phe Ser Met Trp Asn Gln Ala Val Asp Ser Tyr Asp Pro Asp
                   245                 250                 255
   Val Arg Ile Ile Glu Asn His Gly Ile Glu Asp Glu Leu Pro Asn Tyr
               260                 265                 270
   Cys Phe Pro Leu Asp Gly Ile Gly Pro Ala Lys Thr Tyr Gln Gly Ile
           275                 280                 285
   Lys Ser Lys Asp Asn Gly Trp Glu Lys Asp Asp Asn Val Ser Lys Ser
       290                 295                 300
   Asn Glu Ile Ala Ile Gly Asn Asn Gln Ala Met
   305                 310                 315
<210> 13
   <211> 345
   <212> PRT
   <213> Human adenovirus type 2
<400> 13
   Asn Ser Cys Glu Trp Glu Gln Thr Glu Asp Ser Gly Arg Ala Val Ala
   1               5                   10                  15
   Glu Asp Glu Glu Glu Glu Asp Glu Asp Glu Glu Glu Glu Glu Glu Glu
               20                  25                  30
   Gln Asn Ala Arg Asp Gln Ala Thr Lys Lys Thr His Val Tyr Ala Gln
           35                  40                  45
   Ala Pro Leu Ser Gly Glu Thr Leu Thr Lys Ser Gly Leu Gln Ile Gly
       50                  55                  60
   Ser Lys Asn Ala Glu Thr Gln Ala Lys Pro Val Tyr Ala Asp Pro Ser
   65                  70                  75                  80
   Tyr Gln Pro Glu Pro Gln Ile Gly Glu Ser Gln Trp Asn Glu Ala Asp
                   85                  90                  95
   Ala Asn Ala Ala Gly Gly Arg Val Leu Lys Lys Thr Thr Pro Met Lys
               100                 105                 110
   Pro Tyr Gly Ser Tyr Ala Arg Pro Thr Asn Pro Phe Gly Gly Gln Ser
           115                 120                 125
   Val Leu Val Pro Asp Glu Lys Gly Val Pro Leu Pro Lys Val Asp Leu
       130                 135                 140
   Gln Phe Phe Ser Asn Thr Thr Ser Leu Asn Asp Arg Gln Gly Asn Ala
   145                 150                 155                 160
   Thr Lys Pro Lys Val Val Leu Tyr Ser Glu Asp Val Asn Met Glu Thr
                   165                 170                 175
   Pro Asp Thr His Leu Ser Tyr Lys Pro Gly Lys Gly Asp Glu Asn Ser
               180                 185                 190
   Lys Ala Met Leu Gly Gln Gln Ser Met Pro Asn Arg Pro Asn Tyr Ile
           195                 200                 205
   Ala Phe Arg Asp Asn Phe Ile Gly Leu Met Tyr Tyr Asn Ser Thr Gly
       210                 215                 220
   Asn Met Gly Val Leu Ala Gly Gln Ala Ser Gln Leu Asn Ala Val Val
   225                 230                 235                 240
   Asp Leu Gln Asp Arg Asn Thr Glu Leu Ser Tyr Gln Leu Leu Leu Asp
                   245                 250                 255
   Ser Ile Gly Asp Arg Thr Arg Tyr Phe Ser Met Trp Asn Gln Ala Val
               260                 265                 270
   Asp Ser Tyr Asp Pro Asp Val Arg Ile Ile Glu Asn His Gly Thr Glu
           275                 280                 285
   Asp Glu Leu Pro Asn Tyr Cys Phe Pro Leu Gly Gly Ile Gly Val Thr
       290                 295                 300
   Asp Thr Tyr Gln Ala Ile Lys Ala Asn Gly Asn Gly Ser Gly Asp Asn
   305                 310                 315                 320
   Gly Asp Thr Thr Trp Thr Lys Asp Glu Thr Phe Ala Thr Arg Asn Glu
                   325                 330                 335
   Ile Gly Val Gly Asn Asn Phe Ala Met
               340                 345

## Description

This work was funded by grants from the National Institute of Health, P30 DK 47757-08 and P01 HL59407-02 and NIAID grant AI 49766-01. The United States government may have rights in this invention.

### BACKGROUND OF THE INVENTION

Adenoviral recombinants of the human serotype 5 have been tested as vaccine carriers for a variety of antigens derived from viruses, parasites or tumor cells. The results were encouraging as E1-deleted adenoviral recombinants elicited immune responses to the transgene product. Adenovirus of the human serotype 5 (Ad5) is a ubiquitous common-cold virus that infects most humans within their first year of life. The inventors have found that pre-existing immunity to common human serotypes reduce the efficacy of adenoviral recombinant vaccine based on the homologous serotype of virus. In some cases this reduction in efficacy can be overcome by delivery of higher doses of the human adenoviral recombinant. However, these higher doses may be associated with other undesirable side effects.

What are needed are compositions useful for inducing an immune response to a selected molecule, which avoid the problems associated with current delivery methods.

### SUMMARY OF THE INVENTION

The present invention provides means for preferentially inducing a cytotoxic immune response to a heterologous molecule by delivering the molecule to a host via a recombinant simian adenovirus. The inventors have unexpectedly found that recombinant simian adenoviruses, used according to the present invention, present an immunogen in a manner which induces a significantly more potent CD8+ T cell response than when the immunogen is delivered by a comparable human type 5 virus. In addition, the inventors have found that the recombinant chimpanzee adenoviruses induce approximately five-fold higher levels of interferon-α and interferon-β than do the human adenoviruses.

The invention provides, in one aspect, a replication defective simian adenoviral vector containing, in a simian adenoviral capsid, simian adenoviral cis-elements and a heterologous gene operably linked to an expression control sequence, wherein the simian adenoviral capsid is derived from a chimpanzee adenovirus selected from the group consisting of Pan 5, Pan 6, and Pan 7.

The invention also provides a method of producing such a vector, comprising assembling a heterologous gene and the simian adenovirus cis element ITR sequence.

In another aspect, the invention provides the use of a recombinant adenovirus as described, in the preparation of a medicament for inducing a T cell response to an immunogen in a subject, characterized in that said recombinant simian adenovirus preferentially induces a CD8+ T cell response to the immunogen when delivered subcutaneously, and further characterized in that said recombinant adenovirus comprises a nucleic acid molecule encoding an immunogen under the control of regulatory sequences which direct expression of the immunogen in the subject.

In a further aspect, the invention provides the use of a recombinant adenovirus as described, in the preparation of a medicament for inducing an antibody response to an immunogen in a subject, characterized in that said recombinant simian adenovirus preferentially induces an antibody response to the immunogen at low doses when delivered to the musosa, and further characterized in that said recombinant adenovirus comprises a nucleic acid molecule encoding an immunogen under the control of regulatory sequences which direct expression of the immunogen in the subject.

In still another aspect, the invention provides an immunogenic composition useful for inducing a cytolytic immune response for human immunodeficiency virus comprising (a) a recombinant simian adenovirus as described, comprising an optimized nucleic acid sequence encoding a modified gag protein of human immunodeficiency virus-1 and (b) a physiologically compatible carrier.

The invention further provides the use of such an immunogenic composition in preparing a medicament for inducing a CD8+ T cell response against human immunodeficiency virus in mammals.

In a further aspect, the invention provides the use of a recombinant simian adenovirus as described, encoding an immunogenic protein derived from human papilloma virus in preparing a medicament for inducing a CD8+ T cell response against human papilloma virus in mammals.

In an alternative aspect, the invention provides the use of a recombinant simian adenovirus as described, encoding an immunogenic protein derived from rabies virus in preparing a medicament for inducing a neutralizing antibody response against rabies in mammals.

In a further alternative aspect, the invention provides a vaccine for human immunodeficiency virus as described, comprising a recombinant simian replication-defective adenovirus comprising an antigen of human immunodeficiency virus-1 (HIV-1).

In a yet further alternative aspect, the invention provides a vaccine for rabies comprising a recombinant simian replication-defective adenovirus as described, comprising a nucleic acid sequence encoding a rabies antigen under the control of regulatory sequences which direct expression of the rabies antigen.

Yet other advantages of the present invention will be readily apparent from the following detailed description of the invention.

### Brief Description of the Drawings

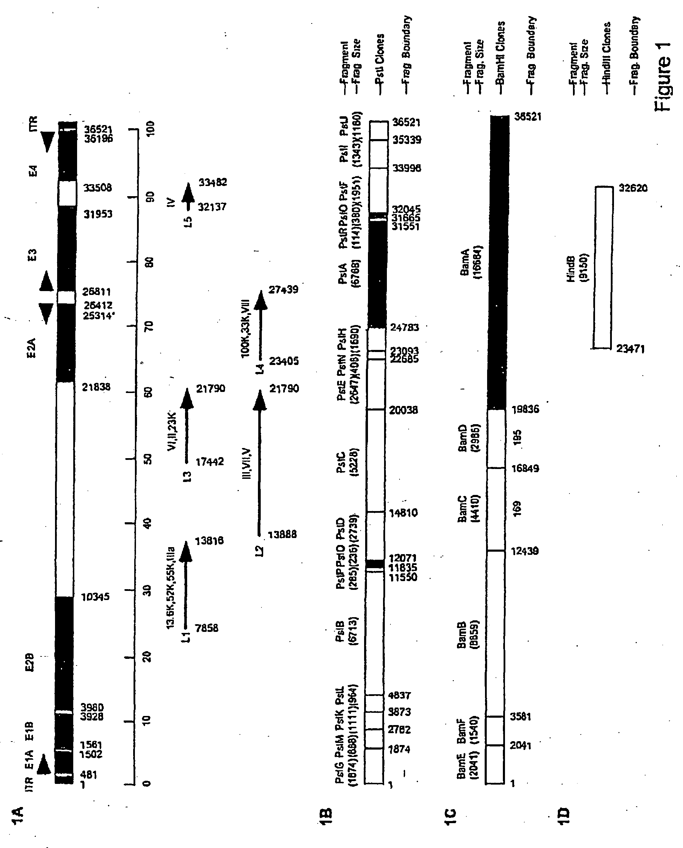

Fig. 1 summarizes the genetic organization of the chimpanzee adenovirus C68 genome. In Fig. 1A the genome of the C68 chimpanzee adenovirus is schematically represented by the box at the top. The inverted terminal repeats are shaded black and the early regions are shaded gray. The arrowheads above the box indicate the direction of expression of the early genes. The line below the box represents the division of the genome into 100 map units. The arrows below the line represent the five late gene regions and the proteins encoded in each region. The numbers below the box or arrows indicate the start (promoter or initiation codon) and end (canonical PolyA signal) for each region. * represents the E2A late promoter. Fig. 1B illustrates the PstI clones; Fig. 1C illustrates the BamHI clones. Fig. 1D illustrates the HindIII clones. For parts 1B-1D, the unshaded regions indicate that a fragment was cloned into a plasmid vector, as listed in Table 1, while the shaded regions indicate that the restriction fragment was not cloned. For each section the fragment name, alphabetical with A being the largest fragment, and the fragment size are listed above the box and the fragment end points are listed below the box.

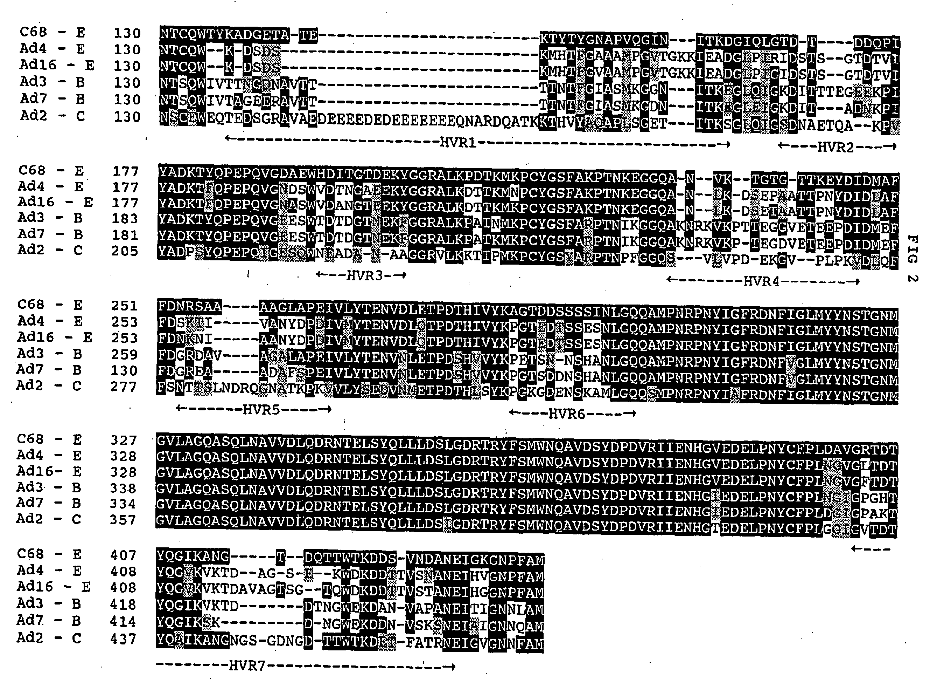

Fig. 2 provides a multiple sequence alignment of hexon proteins. The deduced amino acid sequences of highly similar human adenovirus hexons were compared with the chimpanzee adenovirus using CLUSTAL X. Serotypes and subgroups are indicated on the left margin, followed by the residue number. The numbering refers to the amino acid position with respect to the start of translation. Amino acids are shaded with respect to C68 to highlight sequence similarities (gray) and identities (black). The seven hypervariable regions within loop domains DE1 and FG1 are labeled along the bottom and correspond to the following Ad2 sequences in the alignment: HVR1, 137-188; HVR2, 194-204; HVR3, 222-229; HVR4, 258-271; HVR5, 278-294; HVR6, 316-327; and HVR7, 433-465. The GenBank accession numbers for the sequences shown are as follow: AAD03657 (Ad4), S37216 (Ad16), S39298 (Ad3), AAD03663 (Ad7), and NP040525 (Ad2).

### DETAILED DESCRIPTION OF THE INVENTION

In a comparison of the immunogenicity of the adenoviral recombinants of the human strain 5 to that of the chimpanzee adenovirus strain 68, both expressing a truncated sequence of gag, the chimp adenovirus was shown to be more potent. Similar results have been observed with recombinant chimpanzee adenoviruses expressing the green fluorescent protein and a rabies virus glycoprotein. This higher potency of the recombinant chimpanzee adenovirus is most likely not linked to higher transgene expression as the studies were performed with both Ad and chimp recombinants carrying similar expression cassettes in which the transgene is controlled by the early cytomegalovirus promoter. The data presented herein also indicates that it is unlikely to reflect differences in tropism, as both the chimp and Ad5 viruses utilize the same cellular receptor. Rather, these results demonstrate that the recombinant chimpanzee adenoviruses, used according to the present invention, unexpectedly have an adjuvanticity which differs from the human adenovirus. This better adjuvancy has a profound effect on the magnitude and kinetics of the transgene-specific immune response induced by the chimp adenovirus.

Advantageously, this higher potency permits the use of lower doses of chimpanzee adenoviruses than would be required for a human adenoviral delivery system. In addition, the inventors have found that the recombinant chimpanzee adenoviruses induce approximately five-fold higher levels of interferon-α and interferon-β than do the human adenoviruses.

Further, the recombinant chimpanzee adenoviruses have been found to have approximately the same ability upon dendritic cells as the human adenovirus type 5 viruses. This ability, coupled with the unexpected potency of the simian adenoviruses provides significant advantages in induction of a cytotoxic immune response to a selected antigen and in the treatment of conditions for which enhanced induction of interferon-α and/or interferon-β are desirable.

### I. RECOMBINANT SIMIAN ADENOVIRUS

### A. SOURCES

A variety of sources of chimpanzee adenovirus sequences are available from the American Type Culture Collection, 10801 University Boulevard, Manassas, Virginia 20110-2209, and other sources. Desirable chimpanzee strains Pan 5 [ATCC VR-591], Pan 6 [ATCC VR-592], and Pan 7 [ATCC VR-593]. Particularly desirable chimpanzee adenovirus strains, are chimpanzee adenovirus strain Bertha or C 1 [ATCC Accession No. VR-20] and chimpanzee adenovirus, strain Pan 9 or CV68 [ATCC VR-594]. For convenience, the virus CV68 is referred to throughout this specification as "C68". The viruses were originally isolated from feces [C1, Rowe et al, Proc. Soc. Exp. Med, 91:260 (1956)] or mesenteric lymph node [C68, Basnight et al, Am. J. Epidemiol., 94:166 (1971)] of infected chimpanzees. The sequences of these strains, and the location of the adenovirus genes E1a, E1b, E2a, E2b, E3, E4, L1, L2, L3, L4 and L5 are provided in US Patent 6,083,716, which is incorporated by reference herein. Optionally, non-chimpanzee simian adenoviral sequences may be used in preparing the recombinant vectors of the invention. Such non-chimpanzee adenovirus include those obtained from baboon adenovirus strains [e.g., ATCC VR-275], adenovirus strains isolated from rhesus monkeys [e.g., ATCC VR-209, ATCC VR-275, ATCC VR-353, ATCC VR-355], and adenovirus strains isolated from African green monkeys [e.g., ATCC VR-541; ATCC VR-941; ATCC VR-942; ATCC VR-943].

The recombinant chimpanzee (or other simian) adenoviruses described herein may contain adenoviral sequences derived from one, more than one simian adenoviral strain. These sequences may be obtained from natural sources, produced recombinantly, synthetically, or by other genetic engineering or chemical methods.

### B. RECOMBINANT SIMIAN ADENOVIRUSES

The recombinant simian adenoviruses useful in this invention are viral particles which are composed of recombinant simian adenoviruses sequences carrying a heterologous molecule and/or simian adenovirus capsid proteins. These simian adenoviruses, and particularly the chimpanzee C68 and C1 sequences are also useful in forming hybrid vectors with other simian and non-simian adenoviruses, and in forming pseudotyped recombinant viruses, i.e., recombinant viruses with an adenoviral vector carrying a heterologous molecule which is packaged in a heterologous capsid protein of simian origin.

### 1. Recombinant Simian Adenovirus

At a minimum, a recombinant simian adenovirus useful in the invention contains the simian adenovirus cis-elements necessary for replication and virion encapsidation, which cis-elements flank the heterologous gene. That is, the vector contains the cis-acting 5' inverted terminal repeat (ITR) sequences of the adenoviruses which function as origins of replication), the native 5' packaging/enhancer domains (that contain sequences necessary for packaging linear Ad genomes and enhancer elements for the E1 promoter), the heterologous molecule, and the 5' ITR sequences. See, for example, the techniques described for preparation of a "minimal" human Ad vector in US Patent 6,203,975, which is incorporated by reference, can be readily adapted for the recombinant simian adenovirus.

Optionally, the recombinant simian adenoviruses useful in this invention contain more than the minimal simian adenovirus sequences defined above. These other Ad vectors can be characterized as having modifications which destroy the ability of the adenovirus to express one or more selected gene products. The phrase "functional deletion" is used herein to describe these modifications. Such "functional deletions" typically take the form of a deletion of all or a portion of a gene of the virus. However, such functional deletions may also take the form of a frame shift mutation. Still other suitable manipulations which achieve functional deletion will be readily apparent to those of skill in the art.

In a particularly desired embodiment, the simian adenoviruses are replication defective due to the absence of the ability to express adenoviral E1a and E1b, i.e., are functionally deleted in E1a and Elb. These recombinant simian adenoviruses may also bear functional deletions in other genes.

For example, the adenovirus delayed early gene E3 may be eliminated from the simian adenovirus sequence which forms a part of the recombinant virus. The function of E3 is not necessary to the production of the recombinant adenovirus particle. Thus, it is unnecessary to replace the function of this gene product in order to package a recombinant simian adenovirus useful in the invention.

Recombinant simian adenoviruses may also be constructed having a functional deletion of the E4 gene, although it may be desirable to retain the E4 ORF6 function. Still another vector of this invention contains a deletion in the delayed early gene E2a. Deletions may also be made in any of the late genes L1 through L5 of the simian adenovirus genome. Similarly, deletions in the intermediate genes IX and IVa₂ may be useful for some purposes. Other deletions may be made in the other structural or non-structural adenovirus genes. The above discussed deletions may be used individually, i.e., an adenovirus sequence for use in the present invention may contain deletions of E1 only. Alternatively, deletions of entire genes or portions thereof effective to destroy their biological activity may be used in any combination. For example, in one exemplary vector, the adenovirus sequence may have deletions of the E1 genes and the E4 gene, or of the E1, E2a and E3 genes, or of the E1 and E3 genes, or of E1, E2a and E4 genes, with or without deletion of E3, and so on. Such deletions may be used in combination with other mutations, such as temperature-sensitive mutations, to achieve a desired result.

The transgene may be inserted into any deleted region of the simian adenovirus. Alternatively, the transgene may be inserted into an existing gene region to disrupt the function of that region, if desired.

Regardless of whether the recombinant simian adenovirus contains only the minimal Ad sequences, or the entire Ad genome with only functional deletions in the E1 and/or E3 regions, the recombinant virus contains a simian adenovirus capsid. Alternatively, in other embodiments, recombinant pseudotyped adenoviruses may be used in the methods of the invention. Such pseudotyped adenoviruses utilize simian adenovirus capsid proteins in which a nucleic acid molecule carrying heterologous simian adenovirus sequences, or non-simian adenovirus sequences have been packaged. These recombinant simian adenoviruses of the invention may be produced using methods which are known to those of skill in the art.

### C. Production of the Recombinant Viral Particle

Methods of producing suitable recombinant simian adenoviruses utilize techniques which are well known to those of skill in the art, e.g., such as are described in US Patent 6,083,716. In the construction of recombinant simian adenoviruses for delivery of a heterologous molecule to a subject (e.g., a human, canine, feline, or other mammalian), the adenovirus nucleic acid sequences employed in the vectors can be derived from a variety of simian sources.

A vector comprising simian (e.g., chimpanzee) adenovirus sequences which lacks simian adenovirus sequences necessary for production of an infectious recombinant virus particle may be used in conjunction with a helper virus or vector. The helper virus provides essential gene products required for viral infectivity and propagation of the simian adenovirus. When only one or more selected deletions of simian adenovirus genes are made in an otherwise functional viral vector, the deleted gene products can be supplied in the viral vector production process by propagating the virus in a selected packaging cell.

Thus, these functions may be provided in a permanently transformed cell line which provides some or all of the adenoviral functions which are required for packaging, e.g., any of the E1a, E1b, E2a, E4 ORF6, VA RNAs, which are lacking in the vector. If necessary or alternatively, any additional adenoviral functions required may provided to the packaging cell by transfection or infection of a construct containing these functions. Optionally, the adenoviral functions may be selected to permit packaging of a viral vector carrying the minigene into a heterologous simian adenoviral capsid protein. Suitable methods of "pseudotyping" utilizing the simian (e.g., C68) capsid proteins will be readily apparent based upon that which is known in the art regarding pseudotyping of human adenovirus. See, e.g., US Patent 6,203,975.

Assembly of the selected DNA sequences of the adenovirus, and the transgene and other vector elements into various intermediate plasmids and shuttle vectors, and the use of the plasmids and vectors to produce a recombinant viral particle are all achieved using conventional techniques. Such techniques include conventional cloning techniques of cDNA such as those described in texts [Sambrook et al, Molecular Cloning: A Laboratory Manual, 2nd Ed., Cold Spring Harbor Press, Cold Spring Harbor, NY (1989)], use of overlapping oligonucleotide sequences of the adenovirus genomes, polymerase chain reaction, and any suitable method which provides the desired nucleotide sequence. Standard transfection and co-transfection techniques are employed, e.g., CaPO₄ precipitation techniques. Other conventional methods employed include homologous recombination of the viral genomes, plaquing of viruses in agar overlay, methods of measuring signal generation, and the like.

For example, following the construction and assembly of the desired transgene-containing shuttle vector, the shuttle vector is transfected *in vitro* into the host cell for packaging. The host cell has, or is provided with, any missing adenoviral functions. Homologous recombination occurs between the helper and the vector sequences, which permits the adenovirus-transgene sequences in the vector to be replicated and packaged into virion capsids, resulting in the recombinant adenoviral particles.

Advantageously, the inventors have found that the human adenovirus E1 proteins transcomplement the E1-defective simian adenovirus to permit its packaging into simian adenoviral particles. However, because of the low degree of homology between the human Ad E1 and the sequences flanking the deleted simian Ad E1 sequences, there is minimal risk that the simian Ad E1 will homologously recombine to produce replication-competent simian adenovirus.

The recombinant simian adenoviral particles, so produced, may be isolated and purified by any of a variety of methods known to those of skill in the art for use in the method of the invention.

### II. Heterologous Molecules for Delivery to a Host

### A. Immunogens

The heterologous molecule carried on the simian adenovirus for delivery to a host cell may be any desired substance including, without limitation, a polypeptide, protein, enzyme, carbohydrate, chemical moiety, or nucleic acid molecule which may include oligonucleotides, RNA, DNA, and/or RNA/DNA hybrids. In one desirable embodiment, the molecule carried by the simian adenovirus is a transgene. The transgene a nucleic acid molecule comprising a nucleic acid sequence, heterologous to the adenovirus sequences, which encodes a protein, peptide, polypeptide, enzyme, or another product of interest and regulatory sequences directing transcription and/or translation of the encoded product in a host cell, and which enable expression of the encoded product in the host cell or the subject. The composition of the transgene depends upon the intended use for the simian adenovirus.

For example, one type of transgene comprises a reporter or marker sequence which, upon expression, produces a detectable signal. However, particularly desirable are gene products and other molecules which to which an antibody and, most desirably, cell-mediated immune response are induced.

These immunogenic gene products and molecules may be from a wide variety of pathogenic microorganisms, including but not limited to those from viruses, bacteria, fungi or parasitic microorganisms which infect humans and non-human vertebrates, or from a cancer cell or tumor cell. The immunogen may comprise peptides or polypeptides derived from proteins. In some instances, more than one immunogen is included in the composition.

Desirable immunogenic compositions containing these gene products and other molecules include those directed to the prevention and/or treatment of disease caused by, without limitation, viruses such as Human immunodeficiency virus, Simian immunodeficiency virus, Respiratory syncytial virus, Parainfluenza virus types 1-3, Influenza virus (e.g., influenza A and B viruses), Herpes simplex virus, Human cytomegalovirus, hepatitis viruses (including Hepatitis A, Hepatitis B, and Hepatitis C viruses), Human papillomavirus, poliovirus, rotavirus, caliciviruses, Measles virus, Mumps virus, Rubella virus, adenovirus, rabies virus, canine distemper virus, rinderpest virus, coronavirus, parvovirus, infectious rhinotracheitis viruses, feline leukemia virus, feline infectious peritonitis virus, avian infectious bursal disease virus, Newcastle disease virus, Marek's disease virus, porcine respiratory and reproductive syndrome virus, equine arteritis virus and various Encephalitis viruses.

Still other immunogens are directed to the prevention and/or treatment of disease caused by, without limitation, bacteria such as *Haemophilus influenzae* (both typable and nontypable), *Haemophilus somnus, Moraxella catarrhalis, Streptococcus pneumoniae, Streptococcus pyogenes, Streptococcus agalactiae, Streptococcus faecalis, Helicobacter pylori, Neisseria meningitidis, Neisseria gonorrhoeae, Chlamydia trachomatis, Chlamydia pneumoniae, Chlamydia* psittaci, *Bordetella pertussis, Salmonella typhi, Salmonella typhimurium, Salmonella choleraesuis, Escherichia coli, Shigella, Vibrio cholerae, Corynebacterium diphtheriae, Mycobacterium tuberculosis, Mycobacterium avium-Mycobacterium intracellulare* complex, *Proteus mirabilis, Proteus vulgaris, Staphylococcus aureus, Clostridium tetani, Leptospira interrogans, Borrelia burgdorferi, Pasteurella haemolytica, Pasteurella multocida, Actinobacillus pleuropneumoniae* and *Mycoplasma gallisepticum.*

Still other desirable immunogens are those directed to the prevention and/or treatment of disease caused by, without limitation, fungal pathogens such as *Aspergillis, Blastomyces, Candida, Coccidiodes, Cryptococcus* and *Histoplasma.*

In addition, other desirable immunogens are those directed to the prevention and/or treatment of disease caused by, without limitation, parasites such as *Leishmania major, Ascaris, Trichuris, Giardia, Schistosoma, Cryptosporidium, Trichomonas, Toxoplasma gondii* and *Pneumocystis carinii.*

Further, desirable immunogens include those directed to eliciting a therapeutic or prophylactic anti-cancer effect in a vertebrate host, such as, without limitation, those utilizing a cancer antigen or tumor-associated antigen including, without limitation, prostate specific antigen, carcino-embryonic antigen, MUC-1, Her2, CA-125 and MAGE-3.

The examples provided below specifically illustrate the advantages of the methods and compositions of the invention utilizing a recombinant simian adenoviral vector from which an immunogenic peptide of rabies (glycoprotein G) or human immunodeficiency virus-1 (a modified gag protein) is expressed. Another desirable embodiment utilizes a simian adenovirus carrying an immunogenic peptide from human papilloma virus. However, the invention is not limited to these sources of immunogens.

### B. Regulatory Elements

Design of a transgene or another nucleic acid sequence that requires transcription, translation and/or expression to obtain the desired gene product in cells and hosts may include appropriate sequences that are operably linked to the coding sequences of interest to promote expression of the encoded product. "Operably linked" sequences include both expression control sequences that are contiguous with the nucleic acid sequences of interest and expression control sequences that act in *trans* or at a distance to control the nucleic acid sequences of interest.

Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. A great number of expression control sequences -- native, constitutive, inducible and/or tissue-specific -- are known in the art and may be utilized to drive expression of the gene, depending upon the type of expression desired. For eukaryotic cells, expression control sequences typically include a promoter, an enhancer, such as one derived from an immunoglobulin gene, SV40, cytomegalovirus, etc., and a polyadenylation sequence which may include splice donor and acceptor sites. The polyadenylation (polyA) sequence generally is inserted following the transgene sequences and before the 3' adenovirus ITR sequence. In one embodiment, the bovine growth hormone polyA is selected. A simian adenovirus of the present invention may also contain an intron, desirably located between the promoter/enhancer sequence and the transgene. One possible intron sequence is also derived from SV-40, and is referred to as the SV-40 T intron sequence. Another element that may be used in the vector is an internal ribosome entry site (IRES). An IRES sequence is used to produce more than one polypeptide from a single gene transcript. An IRES sequence would be used to produce a protein that contains more than one polypeptide chain. Selection of these and other common vector elements are conventional and many such sequences are available (see, e.g., Sambrook et al, and references cited therein at, for example, pages 3.18-3.26 and 16.17-16.27 and Ausubel et al., CURRENT PROTOCOLS IN MOLECULAR BIOLOGY, John Wiley & Sons, New York, 1989).

In one embodiment, high-level constitutive expression will be desired. Examples of useful constitutive promoters include, without limitation, the retroviral Rous sarcoma virus (RSV) LTR promoter (optionally with the RSV enhancer), the cytomegalovirus, (CMV) promoter (optionally with the CMV enhancer) (see, e.g., Boshart et al, Cell, 41:521-530 (1985)), the SV40 promoter, the dihydrofolate reductase promoter, the β-actin promoter, the phosphoglycerol kinase (PGK) promoter, and the EF1α promoter (Invitrogen). Inducible promoters, regulated by exogenously supplied compounds, are also useful and include, the zinc-inducible sheep metallothionine (MT) promoter, the dexamethasone (Dex)-inducible mouse mammary tumor virus (MMTV) promoter, the T7 polymerase promoter system (WO 98/10088); the ecdysone insect promoter (No et al, Proc. Natl. Acad Sci. USA, 93:3346-3351 (1996)), the tetracycline-repressible system (Gossen et al, Proc. Natl. Acad Sci. USA, 89:5547-5551 (1992)), the tetracycline-inducible system (Gossen et al, Science, 268:1766-1769 (1995), see also Harvey et al, Curr. Opin. Chem Biol., 2:512-518 (1998)), the RU486-inducible system (Wang et al, Nat. Biotech., 15:239-243 (1997) and Wang et al, Gene Ther., 4:432-441 (1997)) and the rapamycin-inducible system (Magari et al, J. Clin. Invest., 100:2865-2872 (1997)). Other types of inducible promoters which may be useful in this context are those which are regulated by a specific physiological state, e.g., temperature, acute phase, a particular differentiation state of the cell, or in replicating cells only.

In another embodiment, the native promoter for the transgene will be used. The native promoter may be preferred when it is desired that expression of the transgene should mimic the native expression. The native promoter may be used when expression of the transgene must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression.

Another embodiment of the transgene includes a transgene operably linked to a tissue-specific promoter. For instance, if expression in skeletal muscle is desired, a promoter active in muscle should be used. These include the promoters from genes encoding skeletal α-actin, myosin light chain 2A, dystrophin, muscle creatine kinase, as well as synthetic muscle promoters with activities higher than naturally-occurring promoters (see Li et al., Nat. Biotech.,17:241-245 (1999)). Examples of promoters that are tissue-specific are known for liver (albumin, Miyatake et al. J Virol., 71:5124-32 (1997); hepatitis B virus core promoter, Sandig et al., Gene Ther., 3:1002-9 (1996); alpha-fetoprotein (AFP), Arbuthnot et al., Hum. Gene Ther., 7:1503-14 (1996)), bone osteocalcin (Stein et al., Mol. Biol. Rep., 24:185-96 (1997)); bone sialoprotein (Chen et al., J. Bone Miner. Res., 11:654-64 (1996)), lymphocytes (CD2, Hansal et al., J. Immunol., 161:1063-8 (1998); immunoglobulin heavy chain; T cell receptor α chain), neuronal such as neuron-specific enolase (NSE) promoter (Andersen et al. Cell. Mol. Neurobiol., 13:503-15 (1993)), neurofilament light-chain gene (Piccioli et al., Proc. Natl. Acad. Sci. USA, 88:5611-5 (1991)), and the neuron-specific vgf gene (Piccioli et al., Neuron, 15:373-84 (1995)), among others.

Of course, not all expression control sequences will function equally well to express all of the transgenes of this invention. However, one of skill in the art may make a selection among these expression control sequences without departing from the scope of this invention. Suitable promoter/enhancer sequences may be selected by one of skill in the art using the guidance provided by this application. Such selection is a routine matter and is not a limitation of the molecule or construct. For instance, one may select one or more expression control sequences may be operably linked to the coding sequence of interest, and inserted into the transgene, the minigene, and the transfer virus of the invention. After following one of the methods for packaging the simian adenovirus taught in this specification, or as taught in the art, one may infect suitable cells *in vitro* or *in vivo.* The number of copies of the minigene in the cell may be monitored by Southern blotting or quantitative PCR. The level of RNA expression may be monitored by Northern blotting or quantitative RT-PCR. The level of expression may be monitored by Western blotting, immunohistochemistry, ELISA, RIA, or tests of the gene product's biological activity. Thus, one may easily assay whether a particular expression control sequence is suitable for a specific produced encoded by the transgene, and choose the expression control sequence most appropriate. Alternatively, where the molecule for delivery does not require expression, e.g., a carbohydrate, polypeptide, peptide, etc., the expression control sequences need not form part of the recombinant simian adenovirus or other molecule.

### III. FORMULATION OF VIRUS FOR DELIVERY

The recombinant simian adenoviruses, preferably suspended in a physiologically compatible carrier, may be administered to a human or non-human mammalian patient. Suitable carriers may be readily selected by one of skill in the art in view of the indication for which the transfer virus is directed. For example, one suitable carrier includes saline, which may be formulated with a variety of buffering solutions (e.g., phosphate buffered saline). Other exemplary carriers include sterile saline, lactose, sucrose, calcium phosphate, gelatin, dextran, agar, pectin, peanut oil, sesame oil, and water. The selection of the carrier is not a limitation of the present invention.

Optionally, the compositions of the invention may contain, in addition to the recombinant simian adenovirus and carrier(s), other conventional pharmaceutical ingredients, such as preservatives, chemical stabilizers, or for vaccine use, adjuvants. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin. Suitable exemplary adjuvants include, among others, immune-stimulating complexes (ISCOMS), LPS analogs including 3-O-deacylated monophosphoryl lipid A (Ribi Immunochem Research, Inc.; Hamilton, MT), mineral oil and water, aluminum hydroxide, Amphigen, Avirdine, L121/squalene, muramyl peptides, and saponins, such as Quil A.

### IV. DELIVERY OF RECOMBINANT VIRUS FOR TREATMENT AND/OR PROPHYLAXIS

The recombinant, replication defective adenoviruses are administered in a "pharmaceutically effective amount", that is, an amount of recombinant adenovirus that is effective in a route of administration to transfect the desired cells and provide sufficient levels of expression of the selected gene to provide a therapeutic or vaccinal immune response, e.g., some measurable level of protective immunity.

Conventional and pharmaceutically acceptable routes of administration include, but are not limited to, intranasal, intramuscular, intratracheal, subcutaneous, intradermal, rectal, oral and other mucosal and parental routes of administration. As used herein, mucosal routes of administration include those which deliver to mucosal tissues, including, without limitation, inhalation, oral, intranasal, vaginal, and rectal routes. Routes of administration may be combined, if desired, or adjusted depending upon the immunogen or the disease. For example, in prophylaxis of rabies, the subcutaneous, intratracheal, intranasal and oral routes are preferred. The route of administration primarily will depend on the nature of the disease being treated.

Dosses or effective amounts of the recombinant replication-defective Ad virus will depend primarily on factors such as the condition, the selected gene, the age, weight and health of the animal, and may thus vary among animals.

Dosages of the viral vector will depend primarily on factors such as the condition being treated, the age, weight and health of the patient, and may thus vary among mammalian (including human) patients. Advantageously, the unexpected potency of the recombinant simian (e.g., chimpanzee) adenoviruses of the invention permits the use significantly lower amount of the recombinant chimpanzee adenovirus to provide an effective amount to induce the desired immunogenic effect (e.g., induction of a predetermined level of CD8+ T cells). For example, an effective dose of the recombinant simian adenovirus may be provided by 10⁴ pfu and 10⁶ pfu of the chimpanzee adenovirus. However, higher doses may be readily selected, e.g., depending upon the selected route of delivery. For example, the viral vector may be delivered in an amount which ranges from about 100 µL to about 100 ml, and more preferably, about 1 mL to about 10 mL, of carrier solution containing concentrations of ranging from about 1 x 10⁴ plaque forming units (pfu) to about 1 x 10¹³ pfu virus/ml, and about 1 x 10⁹ to about 1 x10¹¹ pfu/ml virus, based upon an 80 kg adult weight. A preferred dosage is estimated to be about 50 ml saline solution at 2 x 10¹⁰ pfu/ml. A preferred dose is from about 1 to about 10 ml carrier (e.g., saline solution) at the above concentrations. The therapeutic levels, or levels of immunity, of the selected gene can be monitored to determine the need, if any, for boosters. Following an assessment of CD8+ T cell response, or optionally, antibody titers, in the serum, optional booster immunizations may be desired. Optionally, the recombinant simian adenoviruses may be delivered using a prime-boost regimen. A variety of such regimens have been described in the art and may be readily selected. One particularly desirable method is described in WO 00/11140, published March 2, 2000, incorporated by reference.

In one desirable embodiment, the invention provides a method of preferentially inducing a CD8+ T cell response to a human immunodeficiency virus in a subject by delivering a recombinant simian adenovirus comprising a modified gag protein. The modified gag protein illustrated in the examples below has been optimized, e.g., as described in US Patent 5,972,596. The coding and protein sequences are reproduced herein in SEQ ID NO:6 and SEQ ID NO:7. See, also, G. Meyers et al., Eds. Human retroviruses and AIDS. A compilation and analysis of nucleic acid and amino acid sequences (Los Alamos National Laboratory, Los Alamos, NM 1991). However, any of a variety of methods to improve expression of the gag protein, or any other selected immunogen or antigen as described herein, are known to those of skill in the art and may be utilized, e.g., humanization of the HIV-1 gag codon sequences, removal of the HIV-1 gag splice site, insertion of additional leader sequences upstream of the HIV-1 gag codon sequences, insertion of a Kozak sequence upstream of the HIV -1 gag codon sequences. The selection of the optimization method is not a limitation of the present invention. Alternatively, the method of the invention may be used to deliver a recombinant simian adenovirus carrying an HIV envelope protein, or an HIV pol, to the subject. One desirable HIV envelope protein is HIV glycoprotein 120 for which sequences are available from GenBank. However, other suitable viral envelope proteins may be utilized. The sequence for HIV-1 pol is known, as are a variety of modified pol sequences. See, e.g., US Patent 5,972,596 and R. Scheider et al, J. Virol, 71 (7):4892-4903 (July 1997).

In another desirable embodiment, the invention provides a method of preferentially inducing a CD8+ T cell response to a tumor-associated protein specific for a selected malignancy by delivering a recombinant simian adenovirus comprising a tumor-associated protein to the subject.. Such a protein includes cellular oncogenes such as mutated ras or p53.

In another embodiment, the invention provides a method of preferentially inducing a CD8+ T cell response to a tumor-associated protein specific for a selected malignancy by delivering a recombinant simian adenovirus comprising a tumor-associated protein to the subject

Still another desirable embodiment involves delivering a recombinant simian adenovirus comprising a protein derived from human papilloma virus for prevention of infection therewith and for treatment and prophylaxis of associated conditions. For example, the protein may be selected from the group consisting of E6, E7 and/or L1 (Seedorf, K. et al, Virol., 145:181-185 (1985)). Where the condition is respiratory syncytial virus infection, the protein is selected from the group consisting of the glyco- (G) protein and the fusion (F) protein, for which sequences are available from GenBank.

The following examples are provided to illustrate the invention and do not limit the scope thereof. One skilled in the art will appreciate that although specific reagents and conditions are outlined in the following examples, modifications can be made which are meant to be encompassed by the spirit and scope of the invention.

### Example 1 - Creation of an E1 deleted vector based on Chimpanzee Adenovirus C68

A replication defective version of C68 was isolated for use in gene transfer. The classic strategy of creating a recombinant with E1 deleted, by homologous recombination in an E1 expressing cell line was pursued. The first step was creation of a plasmid containing m.u. 0 through 1.3 followed by addition of a minigene expressing enhanced green fluorescent protein (GFP) from a CMV promoter and C68 sequence spanning 9-16.7 m.u. This linearized plasmid was cotransfected into an E1 expressing cell line with Ssp I-digested C68 plasmid (SspI cuts at 3.6 m.u. leaving 4644 bp for homologous recombination). Experiments were initially conducted with 293 cells which harbor E1 from human Ad5 with the hope that this would suffice for transcomplementation. Indeed, plaques formed which represented the desired recombinant. The resulting vector was called C68-CMV-GFP.

The strategy for generating recombinants was modified to enable efficient and rapid isolation of recombinants. First, the alkaline phosphatase DNA in the initial shuttle vector was replaced with a prokaryotic GFP gene driven by the prokaryotic promoter from lacZ. This allowed efficient screening of bacterial transformations when attempting to incorporate a desired eukaryotic RNA pol II transcriptional unit into the shuttle vector. The resulting transformation can be screened for expression of GFP; white colonies are recombinants while green colonies are residual parental plasmid.

A green-white selection has been used to screen the products of cotransfection for the isolation of human Ad5 recombinants (A.R. Davis et al, Gene Thera., 5:1148-1152 (1998)); this was adapted to the C68 system. The initial shuttle vector was revised to include extended 3' sequences from 9 to 26 MU. This vector was cotransfected with viral DNA from the original C68-CMV-GFP isolate that had been restricted with Xba I, which cuts at MU 16.5 allowing for 9.5 Kb of overlap for homologous recombination. The resulting plaques were screened under a phase contrast fluorescent microscope for non-fluorescing isolates that represent the desired recombinants. This greatly simplified screening in comparison to the standard methods based on structure or transgene expression.

### A. Shuttle Plasmid

To construct a plasmid shuttle vector for creation of recombinant C68 virus, the plasmid pSP72 (Promega, Madison, WI) was modified by digestion with Bgl II followed by filling-in of the ends with Klenow enzyme (Boehringer Mannheim, Indianapolis, IN) and ligation with a synthetic 12 bp Pac I linker (New England Biolabs, Beverly, MA) to yield pSP72-Pac. A 456 bp Pac I/SnaB I fragment spanning map unit (m.u. or MU) 0-1.3 of the C68 genome was isolated from the pNEB-BamE plasmid containing BamHI E fragment of the C68 genome and cloned into Pac I and EcoR V treated pSP72-Pac to yield pSP-C68-MU 0-1.3. A minigene cassette consisting of the cytomegalovirus early promoter driving lacZ with a SV40 poly A signal was separated from pCMV β (Clontech, Palo Alto, CA) as a 4.5 kb EcoRI/SalI fragment and ligated to pSP-C68-MU 0-1.3 restricted with the same set of enzymes, resulting in pSP-C68-MU 0-1.3-CMVLacZ.

For the initial step in the isolation of the 9-16.7 MU region of C68, both pGEM-3Z (Promega, Madison, MI) and pBS-C68-BamF were double-digested with BamHI and Sph I enzymes. Then the 293 bp fragment from pBS-C68-BamF was ligated with pGEM-3Z backbone to form pGEM-C68-MU 9-9.8. A 2.4 kb fragment including the C68 MU 9.8-16.7 was obtained from the pBS-C68 BamHB clone after XbaI digestion, filling in reaction and subsequent BamHI treatment and cloned into BamHI/SmaI double digested pGEM-C68-MU 9-9.8 to generate pGEM-C68-MU 9-16.7. The C68 9-16.7 m.u. region was isolated from pGEM-C68-MU 9-16.7 by digestion with EcoRI, filling in of the ends with Klenow enzyme (Boehringer Mannheim, Indianapolis, IN), ligation of a synthetic 12 bp HindIII linker (NEB) and then digestion with HindIII. This 2.7 kb fragment spanning the C68 MU 9-16.7 was cloned into the HindIII site of pSP-C68-MU 0-1.3-CMVlacZ to form the final shuttle plasmid pC68-CMV-LacZ. In addition, an 820 bp alkaline phosphatase (AP) cDNA fragment was isolated from pAdCMVALP (K. J. Fisher, et al., J. Virol., 70:520-532 (1996)) and exchanged for lacZ at Not I sites of pC68-CMV-lacZ, resulting in pC68-CMV-AP.

### B. Construction of Recombinant Virus

To create the E1-deleted recombinant C68-CMVEGFP vector, a pC68-CMV-EGFP shuttle plasmid was first constructed by replacing the lacZ transgene in pC68-CMV-lacZ with the enhanced green fluorescent protein (EGFP) gene. The replacement cloning process was carried out as the follows. An additional NotI restriction site was introduced into the 5' end of the EGFP coding sequence in the pEGFP-1 (Clontech, Palo Alto, CA) by BamHI digestion, filling in reaction and ligation of a 8 bp synthetic NotI linker (NEB). After NotI restriction of both constructs, the EGFP sequence was isolated from the modified pEGFP-1 and used to replace the lacZ gene in the pC68-CMV-lacZ. The pC68-CMVEGFP construct (3 µg) was co-transfected with Ssp I-digested C68 genomic DNA (1 µg) into 293 cells for homologous recombination as previously described (G. Gao, et al, J. Virol, 70:8934-8943 (1996)). Green plaques visualized by fluorescent microscopy were isolated for 2 rounds of plaque purification, expansion and purification by CsCl gradient sedimentation (G. Gao, et al, cited above).

In an attempt to apply the convenient green/white selection process (A. R. Davis, et al., Gene Thera, 5:1148-1152 (1998)) to construction of recombinant C68 vectors, a 7.2 kb fragment spanning 9 to 36 MU was isolated from the pBSC68-BamB plasmid by treatment with AgeI and BsiwI restriction endonucleases and cloned into Asp718 and AgeI sites of pC68-CMV-AP shuttle plasmid, resulting in a new plasmid called pC68CMV-AP-MU36. A further modification was made to remove 26 to 36 m.u. from pC68CMV-AP-MU36 by Eco47III and NruI digestions. The new shuttle plasmid called pC68CMV-AP-MU26 has a shorter region for homologous recombination (i.e., 16.7-26 MU) 3' to the minigene. To make a recombinant C68 vector, alkaline phosphatase (AP) is replaced with the gene of interest. The resulting pC68CMV-Nugene-MU26 construct is co-transfected with Xba I (16.5 MU) restricted C68-CMVGFP viral DNA into 293 cells, followed by top agar overlay. The recombinant virus plaques (white) are generated through the homologous recombination in the region of 16.7-26 MU which is shared between pC68CMV-Nugene construct and C68 viral backbone; the recombinants which form white plaques are selected from green plaques of uncut C68-CMVGFP virus.

The green/white selection mechanism was also introduced to the process of cloning of the gene of interest into the pC68 shuttle plasmid. The AP gene in both pC68CMV-AP-MU36 and pC68CMV-AP-MU26 was replaced with a cassette of prokaryotic GFP gene driven by the lacZ promoter isolated from pGFPMU31 (Clontech, Palo Alto, CA). Thus, white colonies of bacterial transformants will contain the recombinant plasmid. This green/white selection process for bacterial colonies circumvented the need for making and characterizing large numbers of minipreped DNAs and so further enhanced the efficiency in creating recombinant C68 vectors.

### Example 2 - Expression of Antigen (Gag secretion) in TK⁻ Cells Infected with Simian Adenovirus Vaccine Constructs

Adenoviral recombinants of the chimpanzee strain 68 (Adchimp68) and the human strain 5 (Adhu5) carrying a nucleotide sequence modified version of a truncated form of the gag gene of HIV-1 clade B were constructed as described (in Example 1 and Z.Q. Xiang, et al, Virol. 219, 200 (1996)). Transcripts of structural proteins of HIV-1, including gag, contain genetic instability elements, which require the presence of rev protein for nuclear export and efficient expression in the cytoplasm (S. Schwartz et al., J Virol 66, 7176 (1992); S.Schwartz, et al, J Virol 66, 150-159 (1992); G. Nasioulas et al., J Virol 68, 2986 (1994)). Adenoviruses rely on nuclear transcription and thus require rev for expression of HIV-1 proteins. To circumvent Rev dependency, a codon-modified sequence of gag from which genetic instability elements had been removed by site directed mutagenesis (R. Schneider et al., J Virol 71, 4892 (1997); S. Schwartz et al., J Virol 66, 7176 (1992); S. Schwartz, et al., J Virol 66, 150 (1992)) was inserted into the adenoviral vector. The introduced gene encodes the truncated p37gag protein (p17 and p24 regions). The truncated gag protein does not form viral particles and is partially secreted into the supernatant of transfected human cells (R. Schneider et al., J Virol 71, 4892 (1997)). The mutated gag constructs have been used in vaccination experiments and result in the generation of cellular and humoral immune responses in mice and primates (J.T. Qiu et al. J Virol 73, 9145. (1999)).

The Adchimp68 and the Adhu5 recombinants were both generated and propagated on 293 cells transfected with the E1 of adenovirus of the human strain 5. The inventors have found that this heterologous E1 is suitable for complementing the E1-deleted Adchimp68 virus recombinants thus reducing the risk of recombination and reversion to replication-competent wild-type virus.

The presence of gag protein in the TK⁻ culture supernatants was analyzed by Western blotting using mouse monoclonal antibodies to gag. TK⁻ cells (1 x 10⁶) were infected for 48 hrs with Adhu5gag37 or Adchimp68gag37 virus (10 pfu per cell). Additional TK⁻ cells were infected with an Adhu5 or an Adchimp68 construct expressing the glycoprotein of rabies virus. Proteins in the culture supernatant were separated on a 12% denaturing polyacrylamide gel and transferred by electroblotting to a PVDF membrane. The blot was stained with the monoclonal antibody 183-H12-5C to HIV-1 p24 (B. Chelsebro, et al. J. Virol. 66: 6547 (1992).

The two adenoviral recombinant clones (Adhu5gag37, Adchimp68gag37) carrying this modified sequence of gag expressed the transgene product at comparable levels as shown by Western Blot analysis. A protein of the expected size (37 kDa) that bound to a monoclonal antibody to gag of HIV-1 was detected in the supernatants of TK⁻ cells infected with 10 plaque forming units (pfu) of either adenovirus gag recombinant. Control cells infected with Adhu5 or Adchimp68 recombinant expressing glycoprotein of rabies virus (Adhusrab.gp and Adchimp68.gp) failed to produce this protein.

### Example 3 - Induction of CD8⁺ T cell responses to gag in mammals by Simian Adenovirus

The following experiment demonstrates that the splenocytes of mice injected intramuscularly (i.m.) with either the Adhu5gag37 or the Adchimp68gag37 recombinant responded to an immunodominant epitope (B. Doe and C.M. Walker, AIDS 10, 793 (1996)) of the gag protein by cytokine, i.e., interferon (IFN)-γ, release, as well as by target cell lysis.

### A. Cytokine Release Assay

Groups of 3 Balb/c mice were immunized i.m. with 2 x 10⁵ , 2 x 10⁶ or 2 x 10⁷ pfu of Adchimp68gag37 virus, 2 x 10⁶ pfu of Adhu5L1 virus (H.C.J. Ertl, et al., J. Virol, 63:2885 (1989)), 2 x 10⁶ pfu of Adhu5 gag37 virus or 2 x 10⁷ pfu of VVgag virus. Splenocytes were tested for CD8⁺ T cell response to gag 10 days later. To assay cytokine (IFN-γ) production, splenocytes (1 x 10⁶/sample) were cultured for 5 hrs at 37°C with 3 µg/ml of the AMQMLKETI peptide (SEQ ID NO:1) which carries the immunodominant CD8⁺ T cell epitope for the H-2^{d} haplotype and 1 µg/ml Brefeldin A (GolgiPlug, PharMingen, San Diego, CA) in 96 well round-bottom microtiter plate wells in Dulbeccos modified Eagles medium (DMEM) supplemented with 2% fetal bovine serum (FBS) and 10⁻⁶M 2-mercaptoethanol. Cells were washed with PBS and incubated for 30 min at 4°C with a FITC labeled antibody to murine CD8. Cells were washed and permeabilized in 1X Cytofix/Cytoperm (PharMingen) for 20 min at 4°C. Cells were washed 3 times with Perm/Wash (PharMingen) and incubated in the same buffer for 30 min at 4 ° C with a PE labeled antibody to murine IFN-γ. After washing, cells were examined by two-color flow cytometry and data were analyzed by WinmDi software. The number in the right hand corner shows the percent of CD8⁺ cells over all CD8⁺ T cells that stained positive for INF-γ.

Seven to ten days after a single immunization, a sizable fraction of the entire splenic CD8⁺ T cell population produced IFN-γ in response to the gag peptide. Primary splenocytes assayed without further in vitro expansion lysed H-2 compatible target cells pre-treated with the gag peptide. Gag-specific CD8⁺ T cell activity was superior upon immunization with the Adchimp68 construct, which achieved CD8⁺ T cell frequencies to gag of ~ 16-19% of the entire splenic CD8⁺ cell population. The response was dose-dependent as shown for the Adchimp68gag37 virus where a low dose of 2 x 10⁵ pfu of virus still elicited frequencies of nearly 10%. The Adhu5gag37 recombinant induced optimal frequencies of ~9% at 2 x 10⁶ pfu. These frequencies were not significantly enhanced upon increasing the dose of this vaccine (data not shown). A vaccinia virus recombinant expressing full-length gag (VVgag, designated vDK1 in S. Chacarabarti et al. Mol. Cell. Biol. 5, 3403 (1985)) stimulated far lower frequencies of CD8⁺ T cells by intracellular cytokine staining.

### B. Lysis of target cells.

Splenocytes from mice immunized 10 days previously with a single dose of the adenoviral recombinants as described in A or two doses of the Wgag recombinant the first given i.m. followed 2 weeks later by an intraperitoneal injection were tested in a 5 hr ⁵¹Cr-release assay at varied effector to target cell ratios on 1 x 10⁴ P815 cells that had been treated for 16-24 hrs at room temperature with either the peptide to gag (filled squares) or the control peptide 31D (X) delineated from the sequence of the rabies virus nucleoprotein (H.C.J. Ertl et al., J. Virol. 63 : 2885 (1989)). Two immunizations with the Wgag vaccine were required to induce detectable T cell-mediated gag-specific primary cytolysis.

### C. Kinetics of the CD8⁺ T cell response to gag

Groups of 4 Balb/c mice were immunized with 5 x 10⁶ pfu of Adhu5gag37 or Adchimp68gag37 virus. Splenocytes were harvested 6-12 days later and tested for IFN-γ production and target cell lysis as described above. The kinetics of the CD8⁺ T cell response to gag elicited by the two adenovirus recombinants differed. The response to gag presented by the Adhu5gag37 virus peaked 2 - 4 days earlier than the CD8⁺ T cell response to the Adchimp68gag37 recombinant.

### Example 4 - Effect of Prior Exposure to Human Adenovirus on Simian Adenovirus Vaccine

To study the impact of previous exposure to the common human strain 5 of adenovirus, mice were immunized with a single dose of an Adhu5 recombinant expressing an irrelevant antigen (human papilloma virus L1). Two weeks later mice were vaccinated either with the Adhu5gag37 or the Adchimp68gag37 vaccine.

More particularly, mice were immunized i.m. with 10⁸ pfu of the Adhu5L1 vaccine. Two weeks later Adhu5-immune as well as naive mice were injected with 2 x 10⁶ or 2 x 10⁷ pfu of Adhu5gag37 or Adchimp68gag37 recombinants (4-5 mice per group). Additional groups of Adhu5L 1 immune or naïve mice were immunized with 2 x 10⁶ pfu of the Adhu5gag37 or the Adchimp68gag37 virus. Nine days later mice were injected intraperitoneally with 10⁶ pfu of a vaccinia virus recombinant expressing full-length gag. Mice were sacrificed five days after the vaccinia virus injection.

Mice pre-immune to Adhu5 virus failed to respond to gag after vaccination with the Adhu5gag37 vaccine. They showed frequencies of CD8⁺ gag-specific T cells similar to those seen in control mice and correspondingly, their splenocytes failed to lyse gag expressing target cells. In contrast, the CD8⁺ T cell response to gag was only slightly decreased in Adhu5-immune mice vaccinated with the Adchimp68gag37 construct. Frequencies of CD8⁺ T cells to gag were reduced by only ~ 30% and the cytolytic activity of splenocytes was lowered by ~ 5.0% comparing different effector to target cell ratios.

Thus, both adenoviral recombinants induce frequencies of CD8⁺ T cells to gag, surpassing those elicited by previously described vaccines such as naked DNA or poxvirus recombinants (S.Schwartz, et al, J Virol 66,150-159 (1992)). Frequencies were also higher than those generally seen in chronically infected individuals (D.H. Barouch et al. Proc. Natl. Acad. Sci USA. 97, 4192 (2000); T.U. Vogel et al. J. Immunol. 164, 4968 (2000); P.A. Goepfert et al. J. Virol. 74, 10249 (2000); C.R. Rinaldo Jr. et al. AIDS Res. & Hum. Retr.. 14:1423 (1998)). These results emphasize the potency of adenoviral recombinant vaccines.

### Example 5 - Effect of Priming and Boosting of CD8⁺ T cells to Antigen

Primary splenocyctes from the cells of naive or Adhu5-immune mice immunized with 2 x 10⁷ pfu of Adhu5gag37 or Adchimp68gag37 virus were compared with splenocytes from naïve or Adhu5 immune mice vaccinated with 2 x 10⁶ pfu of Adhu5gag37 or Adchimp68gag37 virus and then boosted with 10⁶ pfu of VVgag virus. Splenocytes were analyzed 5 days later for CD8 and intracellular IFN-γ. These assays were performed essentially as described above, with the exception that there was no further *in vitro* culture for lysis of P815 cells treated with the gag peptide or the control peptide 31D in a 5 hr ⁵¹Cr-release assay.

Priming or booster immunization with a heterologous vaccine construct, the VVgag recombinant, failed to restore the CD8 T cell response to gag presented by the Adhu5 recombinant vaccine. Although Adhu5 vaccinated animals boosted with Adhu5gagp37 and VVgag showed as much as 7.1 % of splenic CD8⁺ T cells to produce IFN-γ-in response to the gag these CD8⁺ T cells totally lacked cytolytic activity against gag-presenting target cells. These results indicate that pre-exposure to the antigens of the vaccine carrier had not only a quantitative but also a qualitative influence on the CD8⁺ T cell response to the transgene product of the adenoviral recombinant. The CD8⁺ T cell response to gag in Adhu5 immune mice vaccinated with Adchimp68gag37 showed a booster effect upon VVag immunization similar to that seen in naïve mice.

Frequencies of CD8⁺ T cells to gag as well as primary target cell lysis could be augmented further by priming (not shown) or boosting with a heterologous vaccine carrier, such as the VVgag recombinant. After i.m. priming with the adenoviral recombinants followed 9 days later by an i.p. booster immunization with the VVgag, CD8⁺ gag-specific T cells analyzed 5 days post-prime comprised ~ 40% of the entire splenic CD8⁺ cell-population.

Pre-existing immunity to Adhu5 severely reduced the efficacy of the Adhu5gag37 vaccine but only slightly impaired the CD8⁺ T cell response to the Adchimp68gag37 virus. It was previously reported that mice immunized to Adhu5 virus developed a reduced B cell response to vaccination with an Adhu5 vaccine to rabies virus. Increasing the dose of the vaccine or using a DNA vaccine expressing the same antigen of rabies virus could readily circumvent the dampening effect of the pre-exposure to Adhu5 virus (Z.Q. Xiang, et al., J. Immunol. 162, 6716 (1999)).

In contrast, the CD8⁺ T cell response to gag presented by the Adhu5 recombinant vaccine was abolished in Adhu5 immune mice and could only partially be restored by additional immunizations with a heterologous vaccine to gag. This may indicate induction of CD8⁺ T cells to be more susceptible to interference by circulating virus neutralizing antibodies as compared to stimulation of B cells.

### Example 6 - Production of Recombinant Adenoviruses Containing Rabies Glycoprotein

Adenoviruses of the human serotypes 2,4, 5, 7, 12 and the chimpanzee serotype 68 were propagated and titrated on human 293 cells. The recombinant adenoviruses based on the human serotype 5 expressing the glycoprotein of the ERA serotype of rabies virus or the L1 protein of the human papilloma virus (HPV)-16 have been described previously (Z. Q. Xiang, et al, Virology 219: 220-227 (1996); D. W. Kowalcyk, et al, (2001) Vaccine regimen for prevention of sexually transmitted infections with human papillomavirus type 16. Vaccine)*.* An expression system based on adenoviruses of the chimpanzee serotype 68 was developed as described in Example 1.

Adenoviruses were propagated on E1 (derived from the human serotype 5)-transfected 293 cells (F. L. Graham, et. al., J. Gen. Virol. 36: 59-74 (1977)). Viruses were harvested by freeze thawing of the cells. For some experiments virus was purified by CsCl gradient purification. For other experiments, cleared supernatant of the infected cells necrotized through three rounds of freeze thawing was used. Viruses were titrated on 293 cells to determine plaque forming units (pfu).

The adenoviral recombinant of the chimpanzee 68 serotype expressing the rabies virus glycoprotein, termed Adchimp68rab.gp was generated in 293 cells transfected with E1 of adenovirus human serotype 5 as described in detail in this example. Viral clones were initially screened by indirect immunofluorescence with the monoclonal antibody 509-6 to a conformation-dependent epitope of the rabies virus glycoprotein. Upon selection of a stable adenoviral subclone, expression of full-length rabies virus glycoprotein by the Ad.chimp68rab.gp virus in infected TK⁻ cells was confirmed by immunoprecipitation, as described in the following example.

### Example 7 - Expression of the transgene product by the adenoviral recombinants.

This example shows that the Adhu5 virus achieved markedly higher levels of rabies virus glycoprotein expression in TK⁻ cells as compared to the Adchimp68 construct. Transcript levels for this transgene paralleled protein expression indicating that the difference was unrelated to differences in post-translational modifications. TK⁻ cells are CAR positive and rates of transduction by the viral serotypes should thus be comparable.

For use in these experiments, mammalian cells, i.e., baby hamster kidney (BHK)-21 cells, E1-transfected 293 cells and TK⁻ cells, were propagated in Dulbecco's modified Eagle's medium (DMEM) supplemented with glutamine, Napyrovate, non-essential amino acids, HEPES buffer, antibiotic and 10% fetal bovine serum (FBS).

### A. Immunoprecipitation

TK⁻ cells (10⁶ per sample) were infected with 5 pfu per cell of either the rabies virus glycoprotein expressing adenoviral recombinants or control constructs expressing an unrelated viral antigen. After 48 hrs cells were washed twice with sterile phosphate buffered saline (PBS) and then incubated for 90 min in serum-free medium prior to the addition of 20 µl of ³⁵S-labeled cystein and methionin (Promix, NEN, Boston, MA). After 4 hrs incubation, cells were washed with PBS and then treated for 20 min with 1 ml of protease inhibitors containing RIPA buffer. Cells and cell debris were removed from the wells, vortexed briefly and centrifuged for 2 min at 12.000 rpm. The supernatant was incubated for 90 min at 4°C with 15 µl/ml of ascitic fluid containing the 509-6 monoclonal antibody to the rabies virus glycoprotein. Protein Sepharose G was added at 75µl per sample and incubated at 4°C under mild agitation for 30 min. The samples were pelleted by centrifugation and washed 4 times with RIPA buffer. The pellets were resuspended in 80 µl of loading buffer, boiled for 4 min. Samples (20 µl) were then separated over a 12% SDS-polyacrylamide (PAGE) gel in comparison to a molecular weight standard. Gels were dried onto filter papers which were exposed for 48 hrs to a Kodak Scientific Imaging Film (X-Omat Blue XB-1).

The Adchimp68rab.gp recombinant expressed a protein of the expected size that bound to the 509-6 antibody. The precipitate of TK⁻ cells infected with the Adhu5rab.gp virus showed a band of the identical size that was absent in lysates from cells infected with adenoviral recombinants expressing an unrelated transgene product. Expression of the rabies virus glycoprotein was more pronounced in cells infected with the Adhu5rab.gp construct. The difference in expression of the transgene product may reflect pre-translational events such as differences in viral uptake, rate of transcription or transcript stability. Alternatively, translational or post-translational differences such as distinct side chain modifications may result in quantitative differences in serologically detectable protein.

To further distinguish between these two possibilities, the total RNA was isolated from TK⁻ cells infected with either of the adenoviral recombinants. Reverse transcribed mRNA to the rabies virus glycoprotein and a housekeeping gene was amplified by real-time PCR performed as described in part B.

### B. Real time reverse transcription-polymerase chain reaction (PCR)

Confluent monolayers of TK⁻ cells were infected in duplicate samples with 10 pfu of either of the adenoviral recombinants. Cells were isolated 24 hrs later and RNA was extracted with the TRI reagent according to the manufacturer's instructions (Mol. Res. Center, Cincinnati, OH). The RNA was treated with RNAse-free DNAse, purified by phenol extraction and adjusted to 50 ng of RNA per sample. The RNA was reverse transcribed and amplified with the Light Cycler-RNA amplification kit SYBR green (Roche, Mannheim, Germany; Z. He, et al, Virology 270: 146-1617 (2000)) using primers for the rabies virus glycoprotein (SEQ ID Neo:2: 5' AA GCA TTT CCG CCC AAC AC; SEQ ID NO:3: 3' GGT TAG TGG AGC AGT AGG TAG A) and the housekeeping gene glutaraldehyde-3-phosphate dehydrogenase (GAPDH (SEQ ID NO: 4: 5' GGT GAA GGT CGG TGT GAA CGG ATT T; SEQ ID.NO:5: 3' AAT GCC AAA GTT GTC ATG GAT GAC C).

The data in Table 1 provides the results. The data show the mean values for duplicate measurements ± SD.

**Table 1**

| | Relative Transcript Quantity | | |
|---|---|---|---|
| Source of RNA | | | |
| | GAPDH | rab.gp | Ratio |
| | | | (GAPDH/rab.gp) |
| TK⁻, Adhu5rab.gp | 3.2±.2 | 3494±18 | 1082 |
| TK⁻, | 0.52±.01111 | 64±6 | 64 |
| Adchimp68rab.gp | | | |

As shown by this data, the transgene transcripts adjusted to those of the housekeeping gene showed a quantitative difference comparable to that of serologically detectable protein.

In data not provided in this example, two other Adchimp68 recombinants expressing the green fluorescent protein and a codon-modified truncated gag protein of the human immunodeficiency virus-1 were compared to the Adhu5 recombinants expressing the same transgene products showed equivalent protein expression levels in TK⁻ cells. From this it has been concluded that the reduced expression of the rabies virus glycoprotein by the Adchimp68 virus reflects a difference neither in viral uptake nor in rate of transcription, which in both constructs is regulated by the same control elements.

### Example 8 - Immunization of mice using a rabies virus antigen.

The rabies virus-specific antibody response to the Ad.chimp68rab.gp virus was compared to that of the Adhu5rab.gp virus in inbred and outbred strains of mice. Mice were injected with serial dilutions of either of the recombinants given s.c. or i.n. Sera were harvested 14 days later and tested for antibodies to the rabies virus glycoprotein by an ELISA and a virus neutralization assay. Adenoviral recombinants expressing an unrelated transgene, i.e., the gag of HIV-1 (described in the Examples above) were used as controls. These recombinants failed to induce an antibody response to rabies virus detectable by either assay. A more detailed discussion of this study and the results follows.

Female 6-8 week old-C3H/He and C57B1/6 mice were purchased from Jackson Laboratory, Bar Harbor Maine. Outbred ICR mice were purchased from Charles River (Wilmington, MA).

Mice were injected with varied doses of the adenoviruses or the adenoviral recombinants given in 100 µl of saline subcutaneously (s.c.) or in 50 µl intranasally (i.n.). Mice were challenged with rabies virus of the CVS-11 strain given at 10 mean lethal doses (LD₅₀) intracerebrally (i.c.). Rabies virus of the Evelyn Rokitniki-Abelseth (ERA) and the Challenge Virus Standard (CVS)-11 strain were propagated on BHK-21 cells. ERA virus was purified over a sucrose gradient, inactivated by treatment with betapropionolactone and adjusted to a protein concentration of 0.1 mg/ml. CVS-11 virus was titrated on BHK-21 cells and by intracerebral injection of adult ICR mice (Z.Q. Xiang, Z.Q. & H.C. Ertl, J. Virol. Meth. 47: 103-16 (1994)). Upon challenge mice were checked every 24-48 hrs for at least 21 days. They were euthanized once they developed complete hindleg paralysis, which is indicative of terminal rabies virus encephalitis.

The serological assays included enzyme linked immunoadsorbant assay (ELISA), isotype profile of antibodies, and virus neutralization assays.

### A. ELISA

Mice were bled a varied time intervals after immunization by retro-orbital puncture. Sera were prepared and tested for antibodies to rabies virus on plates coated with 0.1 µg/well of inactivated rabies virus. Sera were tested for antibodies to adenovirus on plates coated with 0.1 µg/well of purified E1-deleted adenovirus recombinants to GFP of the human serotype 5 or the chimpanzee serotype 68. ELISAs were performed basically as described before (Z. Q. Xiang, et al, Virology 219, 220-227 (1996)). Plates were coated over night. They were then blocked for 24 hrs with PBS containing 3% of bovine serum albumin (BSA). After washing, sera diluted in PBS-3% BSA were added for 60 min. After washing, a 1:100 dilution of alkaline phosphatase conjugated goat anti mouse Ig (Cappel) was added for 1 hr on ice. After washing, substrate was added for 20-30 min at room temperature. Optical density was read at 405 nm.

### B. Isotypes of antibodies

Isotypes of antibodies to rabies virus were determined by an ELISA on plates coated with inactivated ERA virus using the Calbiochem Hybridoma Subisotyping (LaJolla, CA) kit with some minor previously described modifications (Xiang, Virol, 1996, cited above).

The isotype profile of antibodies to also differed upon s.c. immunization but was comparable upon i.n. application of the two adenoviral vaccines. Both recombinants, upon delivery by either route of inoculation, elicited IgG2a antibodies to the antigen of rabies virus.

Both recombinants upon i.n. immunization and the Adhu5rab.gp vaccine upon s.c. administration induced a pronounced IgG1 response indicative of Th2 help, which was lacking in the response to the Ad.chimp68rab.gp construct given s.c.

### C. Neutralizing Antibodies

Sera were tested for neutralizing antibodies to rabies virus of the CVS-11 strain, which is antigenically closely related to the ERA strain (Z. Q. Xiang, et al, Virology. 214: 398-404 (1996)). A WHO reference serum was used for comparison. Titers are expressed as International Units.

The Adchimp68rab.gp virus given s.c. induced a less potent B cell response to the transgene product as compared to the Adhu5rab.gp construct. The difference in magnitude of the antibody response, which was observed at all time points tested depended on the mouse strain and was less pronounced in outbred ICR than in inbred C3H/He mice. In contrast, upon i.n. immunization both vaccines induced comparable titers of antibodies as determined by ELISA and by virus neutralization assay.

The pronounced Th1 response to the Adchimp68rab.gp recombinant upon s.c. immunization contrasting with the more balanced Th1/Th2 response upon injection of the Adhu5rab.gp argues for a difference in adjuvanticity. Upon application to the airways, the natural route of infection for Adhu5 virus and presumably for Adchimp68 viruses both recombinants induced antibody titers to the transgene product that were comparable in magnitude and in their isotype profile. This suggests that postulated differences in tropism and/or adjuvanticity are tissue dependent, i.e., lacking or less pronounced in the airways as compared to the subcutaneum.

### Example 9 - Preferential Induction of Cytotoxic T Cell Response with Recombinant Chimpanzee Adenovirus

Vaccine-induced protection to rabies virus correlates with virus-neutralizing antibodies (VNAs, F. L. Graham, et. al., J. Gen. virol. 36, 59-74(1977)). The studies with the rabies protein thus focused on stimulation of this arm of the immune system. Throughout all of the experiments, mice were immunized with the Adchimp68rab.gp virus and, in parallel, with the previously described Adrab.gp virus based on the human serotype 5. Within this application, this recombinant is referred to as Adhu5rab.gp virus.

Both adenoviral recombinants induced protection to challenge with rabies virus. C3H/He mice immunized with 5 x 10⁶ pfu of either of the adenoviral recombinants given s.c. remained disease-free when challenged 3 weeks later with 10 mean lethal doses (LD₅₀) of rabies virus of the CVS strain. This rabies virus strain is antigenically closely related to the ERA strain but is more virulent in rodents. At a lower vaccine dose of 5 x 10⁵ pfu, the Adhu5rab.gp virus still provided complete protection while a small percentage of Adchimp68rab.gp-immunized mice succumbed to the infection. Further reduction of the vaccine dose resulted in loss of efficacy of the Adchimp68rab.gp vaccine. Upon i.n. immunization, both vaccines provided complete protection if given at 5 x 10⁵ pfu. At a lower dose of 5 x 10⁴ pfu 50% of mice vaccinated with the Adhu5rab.gp vaccine developed progressive disease while those immunized with this dose of the Adchimp68rab.gp recombinant were protected. All of the mice immunized with adenoviral recombinants of either serotype expressing an unrelated antigen or with 5 x 10³ pfu of either of the adenoviral recombinants to the rabies virus glycoprotein developed a fatal rabies encephalitis.

### Example 10 - The Effect of Pre-Existing Immunity to Different Serotypes of Human Adenoviruses on the Antibody Response to Rabies Virus.

To test if pre-exposure to any of the common serotypes of human adenoviruses (e.g., human serotype 2, 4, 5, 7 and 12) would inhibit the antibody response to the Adchimp68rab.gp vaccine, groups of C3H/He mice were immunized with 4 x 10⁸ pfu of replication-competent adenoviruses of the human serotypes 2, 4, 5, 7 or 12 or the chimpanzee serotype 68 (the latter serotype was E1-deleted). Two weeks later, mice were vaccinated s.c. with either Adhu5rab.gp or Adchimp68rab.gp virus. The Adhu5rab.gp recombinant was used at a dose of 2 x 10⁵ pfu per mouse, the Adchimp68rab.gp recombinant, which given s.c. only induces a marginal antibody response in C3H/He mice at such a low dose was injected at 2 x 10⁷ pfu per mouse. Sera were harvested 2 weeks later and tested for antibodies to the rabies virus glycoprotein by an ELISA. The rabies virus-specific response to Adhu5rab.gp was slightly superior in naive mice to that elicited to the Adchimp68 virus. The response to Adhu5rab.gp virus was completely inhibited in Adhu5 pre-immune mice. Some reduction was also seen in mice pre-immune to adenovirus of the human serotypes 4, 2, 7 and 12. The response was not affected in mice that had been pre-exposed to the Adchimp68 virus. The response to the Adchimp68rab.gp virus was strongly inhibited in mice that were pre-immune to the homologous virus. Mice that had previously encountered adenovirus of the human serotype 2 showed a slight reduction of the antibody response to the rabies virus antigen presented by the Adchimp68 vaccine. Mice inoculated with any of the other serotypes of human adenoviruses developed antibody titers to rabies upon Adchiinp68rab.gp virus that were either similar or increased in magnitude compared to those in mice that were naive prior to vaccination. In particular, mice pre-immune to Adhu5 virus developed higher antibody titers upon vaccination with the Adchimp68rab.gp construct which might reflect the presence of cross-reactive T helper cells that promoted the B cell response to the transgene product.

To further determine if at equal vaccine doses the Adchimp68rab.gp vaccine induced superior antibody titers as compared to the Adhu5rab.gp virus in mice pre-immune to Adhu5 virus, a vaccine titration experiment was conducted. Groups of C3H/He mice were immunized s.c. with 4 x 10⁸ pfu of an E1-deleted adenoviral recombinant to the L1 antigen of HPV-16. Mice were vaccinated 2 weeks later with either Adhu5rab.gp or Adchimp68rab.gp virus given s.c. at varied doses. Mice were bled 2 weeks later and serum antibody titers to rabies virus were determined by an ELISA (not shown) and a virus neutralization assay. Neither assay showed a significant reduction for the antibody response to the Adchimp68rab.gp construct in Adhu5-immune mice. The severity of the reduction of antibody titers to rabies virus presented by the Adhu5 construct in mice pre-immune to the homologous virus depended on the vaccine dose. The antibody response to lower doses of vaccine was more affected than the response to higher vaccine doses. VNA titers were substantially more reduced than the ELISA titers. Titers of VNAs to the highest vaccine dose were halved in mice pre-immune to Adhu5 virus while at the two lower vaccine doses titers were reduced by more than 20 fold. At any of the doses tested, the Adchimp68rab.gp recombinant induced higher VNA titers to rabies in Adhu5 pre-immune mice compared to those achieved by an equal dose of the Adhu5rab.gp vaccine. The detrimental effect of pre-existing immunity to Adhu5 on the efficacy of the Adhu5 vaccine was demonstrated further in a protection experiment. Naïve mice immunized with 2 x 10⁵ pfu of Adhu5rab.gp or Adchimp68rab.gp virus were completely protected to challenge with CVS-11 virus. The majority (65%) of Adhu5 pre-immune mice immunized with this dose of the Adhu5rab.gp vaccine succumbed to a rabies virus infection while those vaccinated with the same dose of the Adchimp68rab.gp virus remained protected. Increasing the dose of the Adhu5rab.gp virus to 2 x 10⁶ pfu per mouse restored the efficacy of the vaccine.

The antibody response to the transgene product expressed by the Adchimp68 recombinant was not affected by-pre-existing immunity to common human adenovirus serotypes, which inhibits the response to the corresponding recombinant of the human serotype 5. Upon pre-immunization with replication-competent viruses, the immune response to the Adhu5rab.gp vaccine was abolished in Adhu5 pre-immune mice and reduced in mice pre-immune to other human serotypes of adenovirus such as 2 and 4. The response to the Adchimp68 recombinant was as expected inhibited in mice pre-immune to the homologous virus. This is not of clinical concern as Adchimp68 virus does not circulate in the human population and common human serotypes do not share neutralizing epitopes with Adchimp68 virus.

Pre-exposure to replication-defective Adhu5 virus also reduced the antibody response to the rabies virus glycoprotein presented by the Adhu5 recombinants although the impact was not as severe as in mice previously infected with replication-competent virus. Sera from mice pre-immune to replication-defective Adhu5 virus developed reduced but readily detectable antibodies to rabies virus upon immunization with the Adhu5rab.gp vaccine. Increasing the dose of the Adhu5rab.gp construct could in part circumvent the impact of pre-existing immunity. Vaccine-induced protection against rabies virus requires VNAs, which were not induced as efficiently in pre-immune mice by the Adhu5 vaccine especially when used at lower doses. In Adhu5 pre-immune mice the VNA response to the Adchimp68rab.gp construct was superior at all-doses tested to that of the Adhu5 vaccine thus more than compensating for the slightly lower potency of this vaccine upon s.c. immunization.

Adchimp68 recombinants thus provide an attractive alternative as a vaccine carrier for use in humans. As shown here they are efficacious even when applied at low doses of 2 x 10⁵ pfu through non invasive routes of administration such as the upper airways. Mucosal immunization by i.n. application has the added advantage of favoring induction of responses of the common mucosal immune system, which is distinct from, albeit interconnected with the central immune system targeted by injected vaccines.

### Example 11 - Chimpanzee C68 Virus Stock and Replication

Examples 11 through 15 which follow provide additional characterization of the chimpanzee C68. It will be appreciated by one of skill in the art that this information can be readily used in the construction of novel recombinant chimpanzee adenoviral constructs.

The C68 virus stock was obtained from ATCC (Rockville, MD) and propagated in 293 cells (ATCC) cultured in DMEM (Sigma, St. Louis, MO) supplemented with 10% fetal calf serum (FCS; Sigma or Hyclone, Logan, UT) and 1% Penicillin-Streptomycin (Sigma). Infection of 293 cells was carried out in DMEM supplemented with 2% FCS for the first 24 hours, after which FCS was added to bring the final concentration to 10%. Infected cells were harvested when 100% of the cells exhibited virus-induced cytopathic effect (CPE), collected, and concentrated by centrifugation. Cell pellets were resuspended in 10mM Tris (pH8.0), and lysed by 3 cycles of freezing and thawing. Virus preparations were obtained following 2 ultra centrifuge steps on cesium chloride density gradients and stocks of virus were diluted to 1 x 10¹² particles/ml in 10mM Tris/100mM NaCl/50% glycerol and stored at -70°C.

### Example 12 - Cloning and sequencing of viral genomic DNA

Genomic DNA was isolated from the purified virus preparation following standard methods and digested with a panel of 16 restriction enzymes following the manufacturer's recommendations. Except as noted, all restriction and modifying enzymes were obtained from Boehringer Mannheim, Indianapolis, IN. Genomic DNA was digested with BamHI, PstI, SalI, HindIII or XbaI and the fragments were subcloned into plasmids (K. L. Berkner and P.A. Sharp, Nucl. Acids Res., 11:6003-20 (1983)). After deproteination, synthetic 10bp PacI linkers (New England Biolabs, Beverly, MA) were double digested with PacI and BamHI, or PstI.

The PstI, BamHI and HindIII clones generated from C68 are illustrated in Figure 1, parts C, D and E, respectively. The fragments indicated by the shaded boxes were not cloned, but the sequence of the entire genome has been determined through sequencing overlapping clones and viral DNA directly (unshaded boxes). The cloned fragments are described in Table 2.

**Table 2. C68 plasmid clones and insert sizes**

| | | | | | |
|---|---|---|---|---|---|
| | | | | | |

| **Construct Name** | **Insert Size (base pairs)** | **Fragment 5' End** | **Fragment 3' End** | **5' End Map Unit** | **3' End Map Unit** |
|---|---|---|---|---|---|
| **Pst-I Fragments** | | | | | |
| | | | | | |
| C68-Pst-A | 6768 | 24784 | 31551 | 67.9% | 86.4% |
| pBS:C68-Pst-B | 6713 | 4838 | 11550 | 13.2% | 31.6% |
| pBS:C68-Pst-C | 5228 | 14811 | 20038 | 40.6% | 54.9% |
| pBS:C68-Pst-D | 2739 | 12072 | 14810 | 33.1% | 40.6% |
| pBS:C68-Pst-E | 2647 | 20039 | 22685 | 54.9% | 32.1% |
| pBS:C68-Pst-F | 1951 | 32046 | 33996 | 87.8% | 93.1% |
| pNEB:C68-Pst-G | 1874 | 1 | 1874 | 0.0% | 5.1% |
| pBS:C68-Pst-H | 1690 | 23094 | 24783 | 63.2% | 67.9% |
| pBS:C68-Pst-I | 1343 | 33997 | 35339 | 93.1% | 96.8% |
| pNEB:C68-Pst-J | 1180 | 35340 | 36519 | 96.8% | 100.0% |
| pBS:C68-Pst-K | 1111 | 2763 | 3873 | 7.6% | 10.6% |
| pBS:C68-Pst-L | 964 | 3874 | 4837 | 10.6% | 13.2% |
| pBS:C68-Pst-M | 888 | 1875 | 2762 | 5.1% | 7.6% |
| pBS:C68-Pst-N | 408 | 22686 | 23093 | 62.1% | 63.2% |
| C68-Pst-O | 380 | 31666 | 32045 | 86.7% | 87.7% |
| pBS:C68-Pst-P | 285 | 11551 | 11835 | 31.6% | 32.4% |
| C68-Pst-Q | 236 | 11836 | 12071 | 32.4% | 33.1% |
| pBS:C68-Pst-R | 114 | 31552 | 31665 | 86.4% | 86.7% |
| | | | | | |

| **BamHI Fragments** | | | | | |
|---|---|---|---|---|---|
| | | | | | |
| C68-Bam-A | 16684 | 19836 | 36519 | 54.3% | 100.0% |
| pBS:C68-Bam-B | 8858 | 3582 | 12439 | 9.8% | 34.1% |
| pBS:C68-Bam-C | 4410 | 12440 | 16849 | 34.1% | 46.1% |
| pBS:C68-Bam-D | 2986 | 16850 | 19835 | 46.1% | 54.3% |
| pNEB:C68-Bam-E | 2041 | | 2041 | 0.0% | 5.6% |
| pBS:C68-Bam-F | 1540 | 2042 | 3581 | 5.6% | 9.8% |
| | | | | | |
| HindIII Fragments | | | | | |
| | | | | | |
| pBR:C68-Hind-B | 9150 | 23471 | 32620 | 64.3% | 89.3% |
| | | | | | |

| | | | | | |
|---|---|---|---|---|---|
| pBS = pBluescript SK+ clone pNEB = pNEB 193 clone pBR = pBR322 clone No prefix = fragment not cloned | | | | | |

Chimpanzee adenovirus, C68, was obtained from ATCC and propagated in human 293 cells. Viral genomic DNA was isolated from purified virions using established procedures (A. R. Davis, et al., Gene Thera., 5:1148-1152 (1998)) and digested with a panel of restriction enzymes; the data were consistent with previous studies (data not shown) (G. R. Kitchingman, Gene, 20:205-210 (1982); Q. Li and G. Wadell, Arch Virol. 101:65-77 (1998); R. Wigand, et al., Intervirology. 30:1-9 (1989)). Restriction fragments spanning the entire genome of C68 were subcloned into plasmids. A schematic drawing of the C68 genome is shown in Figure 1A, and the Pst-I, BamHI and HindIII fragments that were cloned into plasmid vectors are indicated by the unshaded boxes, in Figs. 1B, 1C, and 1D, respectively. The cloned fragments, fragment sizes and genomic position are also listed in Table 2. Both plasmid clones and genomic DNA were used as templates for sequencing. The genome was sequenced by primer walking in both directions and each base was included in an average of approximately four reactions.

The C68 genome is 36521 bp in length [see, US Patent 6,083,716]. Preliminary comparison with GenBank sequences indicated varying degrees of similarity with other human and animal adenoviruses along the entire length of the viral genome. Regions with homology to all of the previously described adenoviral genetic units, early regions 1-4 and the major late genes, were found in the C68 genome (Fig. 1A). DNA homology between C68 and the human adenoviruses that have been completely sequenced, Ad2 (NC001405), Ad5 (NC001405), Ad12. (NC001460), Ad17 (NC002067) and Ad40 (NC01464), was used to order the clones. The open reading frames (ORF) were determined and the genes were identified based on homology to other human adenoviruses. All of the major adenoviral early and late genes are present in C68. The inverted terminal repeats (ITR=s) are 130 bp in length.

### Example 13 - Analysis of C68 sequence

The complete nucleotide sequence of every member of the Mastadenovirus genus accessible from GenBank, including isolates from different species, were screened for identity to C68. The Ad4 minigenome was assembled from the following GenBank sequences: Left-hand ITR (J01964); ERA region (M14918); DNA pol and pTP (X74508, 74672); VA RNA-I, II (U10682); 52, 55K (U52535); pVII (U70921); hexon (X84646); endoprotease (M16692); DNA-binding protein (M12407); fiber (X76547); right-hand ITR (J01965). The Ad7 composite genome was created from the following sequence data: Mu 3-21 (X03000); VA RNA-I, II, pTP & 52, 55K (U52574); penton (AD001675); pVI, hexon and endoprotease (AF065065); DNA-binding protein (K02530); E3 and fiber region (AF104384); right-hand ITR (V00037).

The amino acid sequence alignment was generated with Clustal X, edited with Jalview (http://www.ebi.ac.uk/∼michele/jalview/), and analyzed with Boxshade (http://www.ch.embnet.org/software/BOX_form.html). Publicly available hexon protein sequences from all human adenovirus serotypes were initially aligned to identify the set showing the highest homology to C68.

The nucleotide sequence and predicted amino acid sequences of all significant open reading frames in the C68 genome were compared to known DNA and protein sequences. The nucleotide sequence of C68 is compared to sequences of Ad 2, 4, 5, 7, 12, 17 and 40. In agreement with previous restriction analysis (Kitchingman, cited above; Li and Wadell, cited above) C68 is most similar to human Ad4 (subgroup E).

The E1A region of C68 extends from the TATA box at nt 480 to the poly A addition site at 1521. The consensus splice donor and acceptor sites are in the analogous position of the human Ad counterparts, and the 28.2K and 24.8K proteins are similar in size to the human Ad proteins. The ORF for the smallest E1A protein of C68 is predicted to encode 101 residues as opposed to approximately 60 amino acids for other adenoviruses. There is a TTA codon at residue 60 for C68 where other adenoviruses often have a TGA stop codon. The first 60 residues of C68 E1A 100R protein have 85% identity to the Ad4 homolog.

The C68 genome encodes genes for the four E1B proteins, 20.5K, 54.7K, 10.1K and 18.5K as well as pIX. All five C68 encoded proteins are similar in size to that of other Ad E1B and pIX proteins. The Ad4 homolog of the E1B 21K protein has only 142 amino acids, where C68 has 186 residues and other human adenoviruses have 163-178 residues. The C68 and Ad4 proteins share 95% identity over the first 134 aa, then the similarity ends and the Ad4 protein terminates at 142 amino acids.

The C68 genome encodes homologs of the E2A 55K DNA binding protein and the Iva2 maturation protein, as well as the E2B terminal protein and the DNA polymerase. All of the E2 region proteins are similar in size to their human Ad counterparts, and the E2B proteins are particularly well conserved. The C68 E2B 123.6K DNA polymerase is predicted to be 1124 residues, while Ad4 is predicted to have 1193 although the other human adenoviruses have smaller polymerases. Residues 1-71 of the Ad4 polymerase have no similarity to any other Ad polymerase, and it is possible that this protein actually initiates at an internal ATG codon. From amino acids 72-1193, Ad4 and C68 polymerases have 96% amino acid identity.

The E3 regions of human adenoviruses sequenced so far exhibit considerable sequence and coding capacity variability. Ad40 has five E3 region genes, Ad12 has six, C68 and Ad5 have seven, Ad38 has eight and Ad3 as well as Ad7 (subgroup B human adenoviruses) have nine putative E3 region genes. The Ad4 E3 region has not yet been sequenced. In comparison with the E3 region of Ad35, all 7 E3 gene homologs were identified in the C68 genome (C. F. Basler and M.S. Horwitz, Virology, 215: 165-177 (1996)).

The C68 E4 region has 6 ORFs and each is homologous to proteins in the human Ad5, 12 and 40 E4 region. The E4 nomenclature is confusing because the ORF2 homologs of C68, Ad12 and Ad40 are approximately 130 residues, while in Ad5 there are two ORFs encoding proteins of 64 and 67 residues with homology, respectively, to the amino and carboxy terminal ends of the larger ORF2 proteins. ORF5 has been omitted in our nomenclature because the 5^{th} ORF in the E4 region is homologous to the widely studied ORF6 protein of human Ad5.

The major late promoter and the tri-partite leader sequences of the C68 genome were located. ORFs with the potential to encode the 15 major late proteins were located. All of the C68 late proteins are similar in size to their human Ad counterparts. The percent amino acid identity between chimpanzee and human Ad late proteins varies considerably. The C68 fiber protein is predicted to have 90% amino acid identity with the Ad4 protein, but much less similarity to the other human Ad fiber proteins. The CAR binding site in the fiber knob is present in C68.

### Example 14 - Virus neutralizing antibody assays

Several studies were performed to determine if there is cross-reactivity between type specific antisera of C68 and human adenovirus. The neutralizing activity of sera was tested as follows. Panels of sera from normal human subjects (N=50), rhesus monkeys (N=52) and chimpanzees (N=20) were evaluated for neutralizing antibodies against Ad5 and C68 based vectors using 293 cells as an indicator cell line. Sera collected from individual humans, rhesus monkeys, or chimpanzees were inactivated at 56°C for 30. minutes. A serial dilution of each sample (1:10, 1:20, 1:40, 1:80, 1:160, 1:320 in 100 µl of DMEM containing 10% FCS) was added to equal amounts of H5.010CMVEGFP (1000 PFU/well) or C68CMVEGFP virus and incubated at 4°C for two hrs. One hundred and fifty microliters of the mixture were transferred onto 2 x 10 293 cells in 96 well flat bottom plates. Control wells were infected with equal amounts of virus (without addition of serum). Samples were incubated at 37°C in 5% CO₂ for 48 hrs and examined under a fluorescent microscope. Sample dilutions that showed >50% reduction of green-fluorescent foci as compared to infected controls were scored positive for neutralizing antibodies.

As expected, approximately 35% of normal human subjects demonstrated neutralizing antibody against Ad5, a frequency much higher than observed in sera of rhesus monkeys and chimpanzee. Neutralizing antibody to C68 was observed in 80% of chimpanzee and only 2% of normal human subjects or rhesus monkeys. Titers of neutralizing antibodies in the non-target species were generally low.

To further evaluate cross-reactivity of C68 with human adenovirus vectors, mice were immunized with 2 x 10⁷ plaque forming units (pfu) of Ad 2, 4, 5, 7 and 12 as well as C68. Sera were harvested 2 weeks later and tested for antibodies that neutralized either Ad5 or C68 vectors. Neutralizing antibody to Ad5 vector was only detected in animals immunized with Ad5. Importantly, the only animals with neutralizing antibody to C68 vector were those immunized with C68 vector; none of the human serotypes tested, including Ad4, generated antibodies in mice that neutralized C68 *in vitro.*

Important to the utility of C68 vector in human trials is the absence of neutralizing antibody in the human population. In our study, a screen of 50 normal human subjects failed to detect any significant neutralizing antibodies (>1:10) using the same assay that showed neutralizing antibodies in >50% of chimpanzees. Furthermore, sera of mice immunized with multiple human Ad serotypes including Ad4, did not neutralize infection with C68.

In another study, groups of ten to twenty ICR mice were vaccinated with varied doses of the Adhu5rab.gp or the AdC68rab.gp vaccine given subcutaneously (s.c.), intranasally (i.n.) or orally (per os). Mice were bled 21 days later and viral neutralizing antibody (VNA) titers expressed as international units were determined. Mice were challenged 4 weeks after vaccinated with 10 mean lethal doses of CVS-24 virus applied directly into the central nervous system.

| | VNA Titers (% survival upon challenge) | | | |
|---|---|---|---|---|
| Vaccine Dose | 5x10⁷ | 5x10⁶ | 5x10⁵ | 5x10⁴. |
| Adhu5rab.gp,s.c. | 972(100) | 324 (100) | 108(100) | 12 (100) |
| AdC68rab.gp, s.c. | 240 (100) | 36 (100) | 12(80) | 8(80) |
| Adhu5rab.gp, i.n. | nt | 162 (100) | 162 (100) | 18 (50) |
| AdC68rab.gp, i.n. | nt | 54 (100) | 162 (100) | 18 (50) |
| | 2x10⁷ | 2x10⁶ | 2x10⁵ | 2x10⁴ |
| Adhu5rab.gp, per os | 108 (100) | 54 (88) | 18 (80) | 4(30) |
| AdC68rab.gp, per os | 108 (100) | 36 (78) | 12 (55) | 0.2(0) |

These data demonstrate that the AdC68 construct unexpectedly induces a better protective antibody response at low doses intranasally than human type 5.

### Example 15 - Structural analysis of hexon proteins

The absence of neutralizing antibodies between C68 and human serotypes compelled us to more carefully evaluate structural differences in the regions of hexon presumed to harbor type specific epitopes. Previous studies have suggested that these epitopes are located within the 7 hypervariable regions of hexon determined by Crawford-Miksza and Schnurr (J. Virol, 70:1836-1844 (1996)). A comparison of the amino acid sequences of hexon proteins between C68 and several human adenoviruses is shown in Figure 2. Indeed, C68 is substantially dissimilar in significant regions of these hypervariable sequences. More detailed modeling of the three dimensional structure of hexon of C68 was performed to map the unique sequences. Models of hexon structures from C68 and Ad4 were generated based on the x-ray crystal structures of hexons for Ad2 and Ad5.

The X-ray crystal structures of Ad5 hexon (Protein Data Bank identifier 1RUX) (J. J. Rux and R. M. Burnett, Mol. Ther. 1:18-30 (2000)), and that for Ad2 (F. K. Athappilly, et al, J. Mol. Biol., 242: 430-455 (1994)), have been further refined to yield the current hexon models (Rux and Burnett, to be published elsewhere). Models of the homologous C68 and Ad4 hexons were initially produced using the Swiss-PdbViewer protein-modeling environment (N. Guez and M.C. Peitsch, Electrophoresis, 18:2714-2723 (1997)). Its automated procedure was used to align the C68 and Ad4 hexon amino acid sequences to those of the Ad2 and Ad5 hexon crystal structures. The sequence alignments were used to guide the threading of the model sequences onto the known molecular structures. The side chain positions of residues not seen in the known structures were selected from a library of side chain protomers. These initial molecular models were then manually adjusted to improve the automated alignment by moving gaps to exposed variable regions and by optimizing the packing of side chains. The positions of external loop segments not observed in the Ad2 and Ad5 template structures were either selected from a library of known loop structures or fitted manually. The conformation of each model was further refined by energy minimization using the molecular mechanics program CHARMM (B. R. Brooks, et al, J. Comp. Chem., 4:187-217 (1983)). The structures of these C68 and Ad4 hexon models were then aligned and a new sequence alignment calculated. The differences between the two structurally aligned hexon sequences were used to color images of the homology models. Graphical images prepared within the Swiss-PdbViewer program were exported and rendered with the Persistence of Vision Ray Tracer program (POV-Ray 2000, Version 3.1 g).

While the overall C68 sequence is very similar to that of Ad4 hexon, the differences between the two sequences are primarily focused in the DE1 and FG1 I loops, and these contain all seven hypervariable regions. It is the DE1, FG1, and FG2 loops, each from a different subunit, that intimately associate to form the tower domains at the top of the trimeric molecule. The hexon towers form much of the exterior surface of the virion and are the sites of antibody attachment. As the sides and base of the hexons pack together within the capsid, these regions are shielded from antibody binding and their sequences are conserved. In contrast, the sequences of C68 and Ad4 are quite different in the hexon towers. This immediately explains why antibodies raised to either of these viruses do not cross-react.

## Claims

1. A replication defective simian adenoviral vector containing, in a simian adenoviral capsid, simian adenoviral cis-elements and a heterologous gene operably linked to an expression control sequence, wherein the simian adenoviral capsid is derived from a chimpanzee adenovirus selected from the group consisting of Pan 5, Pan 6, and Pan 7.

2. A simian adenoviral vector as claimed in claim 1 that is replication defective due to the absence of the ability to express adenoviral EIa and EIb.

3. A simian adenoviral vector as claimed in claim 1 or 2, wherein the delayed early gene E3 is eliminated.

4. A simian adenoviral vector as claimed in any of claims 1 to 3, having a functional deletion in the E4 gene.

5. A simian adenoviral vector as claimed in any of claims 1 to 4, which contains a deletion in the delayed early gene E2a.

6. A simian adenoviral vector as claimed in any of claims 1 to 5, having a deletion in any of the late genes L1 to L5 of the simian adenoviral genome.

7. A simian adenoviral vector as claimed in any of claims 1 to 6, wherein the heterologous gene encodes an immunogenic product from a virus selected from the group consisting of Human immunodeficiency virus, Simian immunodeficiency virus, Respiratory syncytial virus, Parainfluenza virus types 1-3, Influenza virus, Herpes simplex virus, Human cytomegalovirus, hepatitis viruses, Human papillomavirus, poliovirus, rotavirus, caliciviruses, Measles virus, Mumps virus, Rubella virus, adenovirus, rabies virus, canine distemper virus, rinderpest virus, coronavirus, parvovirus, infectious rhinotracheitis viruses, feline leukemia virus, feline infectious peritonitis virus, avian infectious bursal disease virus, Newcastle disease virus, Marek's disease virus, porcine respiratory and reproductive syndrome virus, equine arteritis virus and Encephalitis viruses.

8. A simian adenoviral vector as claimed in any of claims 1 to 6, wherein the heterologous gene encodes an immunogenic product from a bacterium selected from the group consisting of *Haemophilus influenzae, Haemophilus somnus, Moraxella catarrhalis, Streptococcus pneumoniae, Streptococcus pyogenes, Streptococcus agalactiae, Streptococcus faecalis, Helicobacter pylori, Neisseria meningitidis, Neisseria gonorrhoeae, Chlamydia trachomatis, Chlamydia pneumoniae, Chlamydia psittaci, Bordetella pertussis, Salmonella typhi, Salmonella typhimurium, Salmonella choleraesuis, Escherichia coli, Shigella, Vibrio cholerae, Corynebacterium diphtheriae, Mycobacterium tuberculosis, Mycobacterium avium, Mycobacterium intracellulare* complex, *Proteus mirabilis, Proteus vulgaris, Staphylococcus aureus, Clostridium tetani, Leptospira interrogans, Borrelia burgdorferi, Pasteurella haemolytica, Pasteurella multocida, Actinobacillus pleuropneumoniae* and *Mycoplasma gallisepticum.*

9. A simian adenoviral vector as claimed in any of claims 1 to 6, wherein the heterologous gene encodes an inumunogenic product from a fungus selected from the group consisting of *Aspergillis, Blastomyces, Candida, Coccidiodes, Cryptococcus* and *Histoplasma.*

10. A simian adenoviral vector as claimed in any of claims 1 to 6, wherein the heterologous gene encodes an immunogenic product from a parasite selected from the group consisting of *Leishmania major, Ascaris, Trichuris, Giardia, Schistosoma, Cryptosporidium, Trichomonas, Toxoplasma gondii* and *Pneumocystis carinii.*

11. A simian adenoviral vector as claimed in any of claims 1 to 6, wherein the heterologous gene is directed to eliciting an anti-cancer effect utilizing a cancer antigen or tumor-associated antigen selected from the group consisting of prostate specific antigen, carcino-embryonic antigen, MUC-1, Her2, CA-125 and MAGE-3.

12. A method of producing a vector as claimed in any of claim 1 to 11, comprising assembling a heterologous gene and the simian adenovirus cis element ITR sequence.

13. Use of a recombinant adenovirus according to any one of claims 1 to 11 in the preparation of a medicament for inducing a T cell response to an immunogen in a subject,
**characterized in that** said recombinant simian adenovirus preferentially induces a CD8+ T cell response to the immunogen when delivered subcutaneously, and
further **characterized in that** said recombinant adenovirus comprises a nucleic acid molecule encoding an immunogen under the control of regulatory sequences which direct expression of the immunogen in the subject.

14. Use of a recombinant adenovirus according to any one of claims 1 to 11 in the preparation of a medicament for inducing an antibody response to an immunogen in a subject,
**characterized in that** said recombinant simian adenovirus preferentially induces an antibody response to the immunogen at low doses when delivered to the mucosa, and
further **characterized in that** said recombinant adenovirus comprises a nucleic acid molecule encoding an immunogen under the control of regulatory sequences which direct expression of the immunogen in the subject.

15. Use according to claim 13 or claim 14, wherein said recombinant simian adenovirus is formulated for subcutaneous administration.

16. Use according to any one of claims 13 to 15, wherein said recombinant simian adenovirus is formulated for administration at a low dose.

17. Use according to any one of claims 13 to 16, wherein said recombinant simian adenovirus is formulated for administration at an effective dose which is between 10⁴ pfu and 10⁶ pfu.

18. Use according to any one of claims 13 to 16, wherein the immunogen is selected from the group consisting of a peptide, polypeptide or protein is derived from a pathogenic virus selected from the group consisting of human immunodeficiency virus-1, human papilloma virus, and rabies.

19. An immunogenic composition useful for inducing a cytolytic immune response for human immunodeficiency virus comprising (a) a recombinant simian adenovirus according to any one of claims 1 to 7 comprising an optimized nucleic acid sequence encoding a modified gag protein of human immunodeficiency virus-1 and (b) a physiologically compatible carrier.

20. Use of an immunogenic composition according to claim 19 in preparing a medicament for inducing a CD8+ T cell response against human immunodeficiency virus in mammals.

21. Use of a recombinant simian adenovirus according to any one of claims 1 to 7 encoding an immunogenic protein derived from human papilloma virus in preparing a medicament for inducing a CD8+ T cell response against human papilloma virus in mammals.

22. Use of a recombinant simian adenovirus according to any one of claims 1 to 7 encoding an immunogenic protein derived from rabies virus in preparing a medicament for inducing a neutralizing antibody response against rabies in mammals.

23. A vaccine for human immunodeficiency virus according to any one of claims 1 to 7 comprising a recombinant simian replication defective adenovirus comprising an antigen of human immunodeficiency virus-1 (HIV-1).

24. The vaccine according to claim 23, wherein the antigen is selected from among the group consisting of the envelope, pol, or gag regions of HTV-1.

25. The vaccine according to claim 23, wherein the antigen comprises a native antigen from which the genetic instability elements have been removed.

26. The vaccine according to claim 23, wherein the antigen is HIV-1 gag cDNA comprising the sequences of SEQ ID NO:6.

27. A vaccine for rabies comprising a recombinant simian replication-defective adenovirus according to any one of claims 1 to 7 comprising a nucleic acid sequence encoding a rabies antigen under the control of regulatory sequences which direct expression of the rabies antigen.

## Patentansprüche

1. Replikationsdefekter-Affen-adenoviraler Vektor beinhaltend, in einem Affen-adenoviralen Kapsid, Affen-adenovirale cis-Elemente und ein mit einer Expressionskontrollsequenz operabel verbundenes heterologes Gen, wobei das Affen-adenovirale Kapsid von einem Schimpansen-Adenovirus ausgewählt aus der Gruppe bestehend aus Pan 5, Pan 6 und Pan 7 stammt.

2. Affen-adenoviraler Vektor nach Anspruch 1, der aufgrund der Abwesenheit der Fähigkeit adenovirales Ela und Elb zu exprimieren, replikationsdefekt ist.

3. Affen-adenoviraler Vektor nach Anspruch 1 oder 2, wobei das spätere frühe (delayed early) Gen E3 eliminiert ist.

4. Affen-adenoviraler Vektor nach einem der Ansprüche 1 bis 3, der eine funktionelle Deletion im E4-Gen aufweist.

5. Affen-adenoviraler Vektor nach einem der Ansprüche 1 bis 4, der eine Deletion im späteren früheren (delayed early) Gen E2a beinhaltet.

6. Affen-adenoviraler Vektor nach einem der Ansprüche 1 bis 5, der eine Deletion in jedem der späten Gene L1 bis L5 des Affen-adenoviralen Genoms aufweist.

7. Affen-adenoviraler Vektor nach einem der Ansprüche 1 bis 6, wobei das heterologe Gen für ein immunogenes Produkt eines Virus ausgewählt aus der Gruppe bestehend aus Humanem Immunodefizienz-Virus, Affen-Immunodefizienz-Virus, Respiratorischem Syncytial-Virus, Parainfluenza-Virus Typ 1 bis 3, Influenza-Virus, Herpes Simplex Virus, Humanes Cytomegalie-Virus, Hepatitis-Viren, Humanem Papillomavirus, Poliovirus, Rotavirus, Caliciviren, Masernvirus, Mumpsvirus, Rötelnvirus, Adenovirus, Tollwutvirus, Hundestaupevirus, Rinderpestvirus, Coronavirus, Parvovirus, infektiöse Rhinotracheitis Viren, Felinem Leukämievirus, Virus der Felinen Infektiösen Peritonitis, Virus der aviären Infektiösen Bursitis, Newcastle Disease-Virus, Marek's Disease-Virus, Virus des Porzinen Respiratorischen und Reproduktiven Syndroms, Virus der equinen Arthritis und Encephalitis-Viren, kodiert.

8. Affen-adenoviraler Vektor nach einem der Ansprüche 1 bis 6, wobei das heterologe Gen für ein immunogenes Produkt aus einem Bakterium ausgewählt aus der Gruppe bestehend aus *Haemophilus influenzae, Haemophilus somnus, Moraxella catarrhalis, Streptococcus pneumoniae, Streptococcus pyogenes, Streptocoecus agalactiae, Streptococcus faecalis, Helicobacter pylori, Neisseria meningitidis, Neisseria gonorrhoeae, Chlamydia trachomatis, Chlamydia pneumoniae, Chlamydia psittaci, Bordetella pertussis, Salmonella typhi, Salmonella typhimurium, Salmonella choleraesuis, Escherichia coli, Shigella, Vibrio cholerae, Corynebacterium diphtheriae, Mycobacterium tuberculosis, Mycobacterium avium, Mycobacterium intracellulare comp*/*ex, Proteus mirabilis, Proteus vulgaris, Staphylococcus aureus, Clostridium tetani, Leptospira interrogans, Borrelia burgdorferi, Pasteurella haemolytica, Pasteurella multocida, Actinobacillus pleuropneumoniae* und *Mycoplasma gallisepticum,* kodiert.

9. Affen-adenoviraler Vektor nach einem der Ansprüche 1 bis 6, wobei das heterologe Gen für ein immunogenes Produkt von einem Pilz ausgewählt aus der Gruppe bestehend aus *Aspergillus, Blastomyces, Candida, Coccidiodes, Cryptococcus* und *Histoplasma,* kodiert.

10. Affen-adenoviraler Vektor nach einem der Ansprüche 1 bis 6, wobei das heterologe Gen für ein immunogenes Produkt eines Parasiten ausgewählt aus der Gruppe bestehend aus *Leishmania major, Ascaris, Trichuris, Giardia, Schistosoma, Cryptosporidium, Trichomonas, Toxoplasma gondii* und *Pneumocystis carinii,* kodiert.

11. Affen-adenoviraler Vektor nach einem der Ansprüche 1. bis 6, wobei das heterologe Gen darauf gerichtet ist, einen Antikrebseffekt unter Verwendung eines Krebsantigens oder Tumors-assoziierten Antigens ausgewählt aus der Gruppe bestehend aus Prostata Spezifischem Antigen, Carcino-embryonalem Antigen, MUC-1, Her2, CA-125 und MAGE-3 hervorzurufen.

12. Verfahren zur Herstellung eines Vektors nach einem der Ansprüche 1 bis 11, umfassend das Zusammensetzen eines heterologen Gens und der eis-Element ITR-Sequenz des Affen-Adenovirus.

13. Verwendung eines rekombinanten Adenovirus nach zumindestens einem der Ansprüche 1 bis 11 zur Herstellung eines Medikaments um eine T-Zell-Antwort auf ein Immunogen in einem Subjekt zu induzieren,
**dadurch gekennzeichnet, dass** dieses rekombinante Affen-Adenovirus bevorzugt eine CD8⁺-T-Zell-Antwort auf das Immunogen induziert, wenn es subkutan appliziert wird, und
weiter **dadurch gekennzeichnet, dass** dieses rekombinante Adenovirus ein Nukleinsäuremolekül umfaßt, kodierend für ein Immunogen unter der Kontrolle von regulatorischen Sequenzen, die die Expression des Immunogens in dem Subjekt steuern.

14. Verwendung eines rekombinanten Adenovirus nach zumindestens einem der Ansprüche 1 bis 11 zur Herstellung eines Medikaments, um eine Antikörperantwort auf ein Immunogen in einem Subjekt zu induzieren,
**dadurch gekennzeichnet, dass** dieses rekombinante Affen-Adenovirus bevorzugt in niedrigen Dosen eine Antikörperantwort auf das Immunogen induziert, wenn es auf die Schleimhaut verbracht wird, und
weiter **dadurch gekennzeichnet, dass** dieses rekombinante Adenovirus ein Nukleinsäuremolekül umfasst, kodierend für ein Immunogen unter der Kontrolle von regulatorischen Sequenzen, die die Expression des Immunogens in dem Subjekt steuern.

15. Verwendung nach Anspruch 13 oder Anspruch 14, wobei dieses rekombinante Affen-Adenovirus für die subkutane Verabreichung formuliert ist.

16. Verwendung nach zumindestens einem der Ansprüche 13 bis 15, wobei dieses rekombinante Affen-Adenovirus für die Verabreichung in einer niedrigen Dosis formuliert ist.

17. Verwendung nach zumindestens einem der Ansprüche 13 bis 16, wobei dieses rekombinante Affen-Adenovirus für eine Verabreichung von einer effektiven Dosis, die zwischen 10⁴ pfu und 10⁶ pfu liegt, formuliert ist.

18. Verwendung nach zumindestens einem der Ansprüche 13 bis 16, wobei das Immunogen ausgewählt ist aus der Gruppe bestehend aus einem von einem pathogenen Virus stammenden Peptid, Polypeptid oder Protein ausgewählt aus der Gruppe bestehend aus Humanem Immunodefizienz-Virus-1, Humanem Papilloma-Virus, und Tollwut.

19. Immunogene Zusammensetzung nützlich, um eine zytologische Immunantwort für Humanes Immunodefizienz-Virus zu induzieren, umfassend (a) ein rekombinantes Affen-Adenovirus nach zumindestens einem der Ansprüche 1 bis 7 umfassend eine optimierte Nukleinsäuresequenz kodierend für ein modifiziertes gag-Protein von Humanem Immunodefizienz-Virus-1 und (b) einen physiologisch kompatiblen Träger.

20. Verwendung einer immunogenes Zusammensetzung nach Anspruch 19 zur Herstellung eines Medikaments, um eine CD8⁺-T-Zell-Antwort gegenüber Humanem Immunodefizienz-Virus in Säugetieren zu induzieren.

21. Verwendung eines rekombinanten Affen-Adenovirus nach zumindestens einem der Ansprüche 1 bis 7 kodierend für ein immunogenes vom humanen Papillomavirus stammenden Proteins zur Herstellung eines Medikaments zum Induzieren einer CD8+ -T-Zell-Antwort gegen Humanem Papillomavirus in Säugetieren.

22. Verwendung eines rekombinanten Affen-Adenovirus nach zumindestens einem der Ansprüche 1 bis 7 kodierend für ein immunogenes vom Tollwutvirus stammenden Proteins für die Herstellung eines Medikaments, um eine neutralisierende Antikörperantwort gegen Tollwut in Säugetieren zu induzieren.

23. Impfstoff gegen Humanes Immunodefizienzvirus nach einem der Ansprüche 1 bis 7, umfassend ein rekombinantes replikationsdefektes Affen-Adenovirus umfassend ein Antigen vom Humanen Immunodefizienz-Virus-1 (HIV-1).

24. Impfstoff nach Anspruch 23, wobei das Antigen ausgewählt ist aus der Gruppe bestehend aus den envelope-, pol-, oder gag-Regionen von HIV-1.

25. Impfstoff nach Anspruch 23, wobei das Antigen ein natives Antigen umfasst, von dem die Elemente für die genetische Instabilität entfernt worden sind.

26. Impfstoff nach Anspruch 23, wobei das Antigen die HIV-1 gag-cDNA ist, umfassend die Sequenzen von SEQ. ID No: 6.

27. Impfstoff gegen Tollwut umfassend einen rekombinanten replikationsdefekten Affen-Adenovirus nach zumindestens einem der Ansprüche 1 bis 7, umfassend eine Nukleinsäuresequenz kodierend für ein Tollwutantigen unter der Kontrolle von regulatorischen Sequenzen, die die Expression des Tollwutantigens steuern.

## Revendications

1. Vecteur adénoviral simien défectueux en réplication contenant, dans une capside adénovirale simienne, des cis-éléments adénoviraux simiens et un gène hétérologue lié de façon fonctionnelle à une séquence de contrôle d'expression, la capside adénovirale simienne étant issue d'un adénovirus de chimpanzé choisi dans le groupe constitué par Pan 5, Pan 6 et Pan 7.

2. Vecteur adénoviral simien selon la revendication 1, qui est défectueux en réplication en raison de l'absence de l'aptitude à exprimer Ela et Elb adénoviraux.

3. Vecteur adénoviral simien selon l'une des revendications 1 ou 2, dans lequel le gène précoce retardé E3 est éliminé.

4. Vecteur adénoviral simien selon l'une quelconque des revendications 1 à 3, ayant une délétion fonctionnelle dans le gène E4.

5. Vecteur adénoviral simien selon l'une quelconque des revendications 1 à 4, qui contient une délétion dans le gène précoce retardé E2a.

6. Vecteur adénoviral simien selon l'une quelconque des revendications 1 à 5, ayant une délétion dans n'importe lequel des gènes tarifs L1 à L5 du génome adénoviral simien.

7. Vecteur adénoviral simien selon l'une quelconque des revendications 1 à 6, dans lequel le gène hétérologue code pour un produit immunogène provenant d'un virus choisi dans le groupe constitué par le virus de l'immunodéficience humaine, le virus de l'immunodéficience simienne, le virus syncytial respiratoire, les virus de parainfluenza de types 1-3, le virus de la grippe, le virus herpes simplex, le cytomégalovirus humain, les virus de l'hépatite, le papillomavirus humain, le poliovirus, le rotavirus, les calicivirus, le virus de la rougeole, le virus des oreillons, le virus de la rubéole, l'adénovirus, le virus de la rage, le virus de la maladie de Carré, le virus de la peste bovine, le coronavirus, le parvovirus, les virus de la rhinotrachéite infectieuse, le virus de la leucémie féline, le virus de la péritonite infectieuse féline, le virus de la bursite infectieuse aviaire, le virus de la maladie de Newcastle, le virus de la maladie de Marek, le virus du syndrome respiratoire et reproductif porcin, le virus de l'artérite équine et les virus de l'encéphalite.

8. Vecteur adénoviral simien selon l'une quelconque des revendications 1 à 6, dans lequel le gène hétérologue code pour un produit immunogène provenant d'une bactérie choisie dans le groupe constitué par *Haemophilus Influenzae, Haemophilus somnus*, *Moraxella catarrhalis, Streptococcus pneumoniae, Streptococcus pyogenes, Streptococcus agalactiae, Streptococcus faecalis Helicobacter pylori, Neisseria meningitidis, Neisseria gonorrhoeae, Chlamydia trachomatis, Chlamydia pneumoniae*, *Chlamydia psittaci, Bordetella pertussis, Salmonella typhi, Salmonella typhimurium, Salmonella choleraesuis, Escherichia coli, Shigella, Vibrio cholerae, Corynebacterium diphtheriae, Mycobacterium tuberculosis, Mycobacterium avium*, *Mycobacterium intracellulare complex, Proteus mirabilis, Proteus vulgaris, Staphylococcus aureus, Clostridium tetani, Leptospira interrogans, Borrelia burgdorferi*, *Pasteurella haemolytica, Pasteurella multocida, Actinobacillus pleuropneumoniae* et *Mycoplasma gallisepticum.*

9. Vecteur adénoviral simien selon l'une quelconque des revendications 1 à 6, dans lequel le gène hétérologue code pour un produit immunogène provenant d'un champignon choisi dans le groupe constitué par *Aspergillis*, *Blastomyces, Candida, Coccidiodes, Cryptococcus* et *Histoplasma.*

10. Vecteur adénoviral simien selon l'une quelconque des revendications 1 à 6, dans lequel le gène hétérologue code pour un produit immunogène provenant d'un parasite choisi dans le groupe constitué par *Leishmania major, Ascaris*, *Trichuris, Giardia*, *Schistosoma*, *Cryptosporidium, Trichomonas, Toxoplasma gondii* et *Pneumocystis carinii.*

11. Vecteur adénoviral simien selon l'une quelconque des revendications 1 à 6, dans lequel le gène hétérologue est dirigé vers le déclenchement d'un effet anti-cancer utilisant un antigène du cancer ou un antigène associé à une tumeur choisi dans le groupe constitué par un antigène spécifique de la prostate, un antigène carcino-embryonnaire, MUC-1, Her2, CA-125 et MAGE-3.

12. Procédé de production d'un vecteur tel que défini à l'une quelconque des revendications 1 à 11, comprenant l'assemblage d'un gène hétérologue et la séquence ITR de l'élément cis de l'adénovirus simien.

13. Utilisation d'un adénovirus recombinant tel que défini à l'une quelconque des revendications 1 à 11, dans la préparation d'un médicament pour induire une réponse des lymphocytes T à un dans un sujet, 1 **caractérisée par le fait que** ledit adénovirus simien recombinant induit de préférence une réponse des lymphocytes T CD8+ à l'immunogène lorsqu'il est administré par voie sous-cutanée, et
**caractérisée en outre par le fait que** ledit adénovirus recombinant comprend une molécule d'acide nucléique codant pour un immunogène sous le contrôle de séquences régulatrices qui dirigent expression de l'immunogène dans le sujet.

14. Utilisation d'un adénovirus recombinant tel que défini à l'une quelconque des revendications 1 à 11, dans la préparation d'un médicament pour induire une réponse d'anticorps à un immunogène dans un sujet,
**caractérisée par le fait que** ledit adénovirus simien recombinant induit de préférence une réponse d'anticorps à l'immunogène à de faibles doses lorsqu'il est administré à la moqueuse, et
**caractérisée en outre par le fait que** ledit adénovirus recombinant comprend une molécule d'acide nucléique codant pour un immunogène sous le contrôle de séquences régulatrices qui dirigent l'expression de l'immunogène dans le sujet.

15. Utilisation selon a revendication 13 ou la revendication 14, dans laquelle ledit adénovirus simien recombinant est formulé pour une administration sous-cutanée.

16. Utilisation selon l'une quelconque des revendications 13 à 15, dans laquelle ledit adénovirus simien recombinant est formulé pour une administration à une faible dose.

17. Utilisation selon l'une quelconque des revendications 13 à 16, dans laquelle ledit adénovirus simien recombinant est formulé pour une administration à une faible efficace qui est entre 10⁴ pfu et 10⁵ pfu.

18. Utilisation selon l'une quelconque des revendications 13 à 16, dans laquelle l'immunogène est choisi dans le groupe constitué par un peptide, un polypeptide ou une protéine qui est issu d'un virus pathogène choisi dans le groupe constitué par le virus de l'immunodéficience humaine-1, le virus du papillome humain et la rage.

19. Composition immunogène utile pour induire une réponse immunitaire cytolytique pour le virus de l'immunodéficience humaine comprenant (a) un adénovirus simien recombinant tel que défini à l'une quelconque des revendications 1 à 7, comprenant une séquence d'acide nucléique optimisée codant pour une protéine gag modifiée du virus de l'immunodéficience humaine-1 et (b) un support physiologiquement compatible.

20. Utilisation d'une composition immunogène tel que défini à la revendication 19, dans la préparation d'un médicament pour induire une réponse des lymphocytes T CD8+ à rencontre du virus de l'immunodéficience humaine dans des mammifères.

21. Utilisation d'un adénovirus simien recombinant tel que défini à l'une quelconque des revendications 1 à 7, codant pour une protéine immunogène issue du virus du papillome humain dans la préparation d'un médicament pour induire une réponse des lymphocytes T CD8+ à l'encontre du virus du papillome humain dans des mammifères.

22. Utilisation d'un adénovirus simien recombinant tel que défini à l'une quelconque des revendications 1 à 7, codant pour une protéine immunogène issue du virus de la rage dans la préparation d'un médicament pour induire une réponse d'anticorps neutralisant à l'encontre de la rage dans des mammifères.

23. Vaccin pour le virus de l'immunodéficience humaine tel que défini à l'une quelconque des revendications 1 à 7, comprenant un adénovirus simien recombinant défectueux en réplication comprenant un antigène du virus de l'immunodéficience humaine-1 (VIH-1).

24. Vaccin selon la revendication 23, dans lequel l'antigène est choisi dans le groupe constitué par les régions d'enveloppe, pol ou gag du VIH-1.

25. Vaccin selon la revendication 23, dans lequel l'antigène comprend un antigène natif duquel les éléments d'instabilité génétique ont été retirés.

26. Vaccin selon la revendication 23, dans lequel l'antigène est l'ADNc de gag du VIH-1 comprenant les séquences de SEQ ID N° 6.

27. Vaccin pour la rage comprenant un adénovirus simien recombinant défectueux en réplication tel que défini à l'une quelconque des revendications 1 à 7, comprenant une séquence d'acide nucléique codant pour un antigène de la rage sous le contrôle de séquences régulatrices qui dirigent l'expression de l'antigène de la rage.
